(19)

Europäisches Patentamt
European Patent Office
Office européen des brevets

(11) **EP 4 537 783 A1**

(12) **EUROPEAN PATENT APPLICATION**
published in accordance with Art. 153(4) EPC

(43) Date of publication:
**16.04.2025 Bulletin 2025/16**

(21) Application number: **23818876.7**

(22) Date of filing: **10.05.2023**

(51) International Patent Classification (IPC):
*A61B 34/37* (2016.01)

(52) Cooperative Patent Classification (CPC):
**A61B 34/37**

(86) International application number:
**PCT/CN2023/093226**

(87) International publication number:
**WO 2023/236711 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.06.2022 CN 202210656808**

(71) Applicant: **Shenzhen Edge Medical Co., Ltd. Shenzhen, Guangdong 518000 (CN)**

(72) Inventors:
• LIU, Fang
  **Shenzhen, Guangdong 518000 (CN)**
• WANG, Jianchen
  **Shenzhen, Guangdong 518000 (CN)**

(74) Representative: **Zaboliene, Reda Metida Business center Vertas Gyneju str. 16 01109 Vilnius (LT)**

(54) **INPUT DEVICE AND SURGICAL ROBOT**

(57)     The application relates to an input device and a surgical robot. The input device includes a base, a first arm, a second arm, a first motor, a first transmission mechanism, and an operating assembly. The base, the first arm, the second arm, and the operating assembly are sequentially and rotatably connected to each other. The operating assembly can receive operations of an operator. The first arm and the second arm can rotate to allow the operating assembly to move in at least two degrees of freedom. The first motor is mounted on the base, and the first arm includes an arm turntable. The arm turntable is rotatably connected to an output shaft of the first motor through the first transmission mechanism. The rotation of the output shaft drives the arm turntable to rotate, such that the first arm rotates relative to the base. A rotational speed of the output shaft is greater than that of the arm turntable.

FIG. 8A

## Description

The present application claims priority to Chinese Patent Application No. 202210656808.X, filed to the China National Intellectual Property Administration on June 11, 2022, and entitled "INPUT DEVICE AND SURGICAL ROBOT".

FIELD

The present invention relates to the field of robot technologies, and more particularly, to an input device and a surgical robot.

BACKGROUND

Minimally invasive surgery refers to a surgical method that uses modern medical instruments such as laparoscope and thoracoscope and related equipment to perform surgery inside the human body cavity. Compared to existing surgical methods, minimally invasive surgery has advantages of less trauma, less pain, and faster recovery.

Taking a laparoscopic surgical robot as an example, the laparoscopic surgical robot includes a master console and a slave operating device controlled by the master console. The master console includes an input device, and doctors may control the slave operating device through control commands, which are generated by operating the input device.

In some existing products, the input device has a large size, resulting in poor operation flexibility.

SUMMARY

The main purpose of the present invention is to provide an input device and a surgical robot, which may solve the problems of large size of the input device size and poor operation flexibility.

The present application provides an input device, including a base, a first arm, a second arm, a first motor, a first transmission mechanism, and an operating assembly. The base, the first arm, the second arm, and the operating assembly are sequentially and rotatably connected to each other. The operating assembly is configured to receive operations of an operator, the first arm and the second arm are configured to rotate to allow the operating assembly to move in at least two degrees of freedom. The first motor is mounted on the base. The first arm includes an arm turntable. The arm turntable is rotatably connected to an output shaft of the first motor through the first transmission mechanism. The output shaft is configured to rotate to drive the arm turntable to rotate, such that the first arm rotates relative to the base. A rotation speed of the output shaft is greater than a rotation speed of the arm turntable.

Wherein, the first transmission mechanism includes a first transmission member, the first transmission member is connected between the arm turntable and the output shaft of the first motor, and radius of the arm turntable is greater than a radius of the output shaft of the first motor.

Wherein, the first transmission mechanism includes a first transmission member, a first adjusting member, and a second transmission member, the first transmission member is rotatably connected between the output shaft and an input portion of the first adjusting member, the second transmission member is connected between an output portion of the first adjusting member and the arm turntable, and a radius of the input portion is larger than a radius of the output portion.

Wherein, the input device further includes a second motor and a second transmission mechanism, the second motor is mounted on the base, and an output shaft of the second motor is rotatably connected to the second arm through the second transmission mechanism.

Wherein, the input device further includes a transmission turntable and a transmission arm, the transmission turntable is rotatably connected to the base, the transmission arm is rotatably connected between the transmission turntable and the second arm, the second motor is configured to drive the transmission turntable to rotate through the second transmission mechanism, the transmission turntable is configured to drive the transmission arm to move when the transmission turntable rotates, such that the second arm rotates relative to the first arm.

Wherein, the base includes a main body, a first mounting plate, and a second mounting plate, the first mounting plate and the second mounting plate are connected to the main body and opposite to each other, the arm turntable and the first motor are mounted on the first mounting plate and spaced from each other, and the transmission turntable and the second motor are mounted on the second mounting plate and spaced from each other.

Wherein, the second motor is parallel to the first motor.

Wherein, one end of the first motor near the second mounting plate extends through the second mounting plate. Wherein, one end of the second motor near the first mounting plate extends through the first mounting plate.

Wherein, the input device further includes a fixing shaft, the fixing shaft extends through and is fixed to each of the first mounting plate and the second mounting plate, and two ends of the fixing shaft are rotatably connected to the arm turntable and the transmission turntable, respectively.

Wherein, the input device further includes a connecting member, a main motor, and a main transmission mechanism, the base further includes a main turntable, the main turntable is connected to the main body, the main motor is mounted on the connecting member, and an output shaft of the main motor is rotatably connected to the main turntable through the main transmission mechanism.

Wherein, an extension line of the main motor in a length direction thereof is perpendicular to a rotation axis of the arm turntable, and an extension line of each of the first motor and the second motor in a length direction thereof is parallel to the rotation axis of the arm turntable.

Wherein, the input device further includes a first gravity compensation mechanism connected between the base and the first arm, and the first gravity compensation mechanism is configured to generate a force moment to balance a gravity moment of relevant components of the input device.

Wherein, the first gravity compensation mechanism includes a plurality of rotating portions, a first cable, and a first elastic element, at least a portion of the rotating portions is fixed to the arm turntable, an end of the first elastic element is fixed to the base, another end of the first elastic element is fixed to the first cable, an end of the first cable away from the first elastic element is engaged with the rotating portions and fixed to one of the rotating portions.

Wherein, the input device further includes a first sensor, the first sensor is configured to sense position change of the first arm, and the first motor is configured to cooperate with the first gravity compensation mechanism to generate the force moment to balance the gravity moment of the relevant components of the input device.

The present application further includes an input device, including:

> a base;
> a first arm rotatably connected to the base;
> a first motor, an output shaft of the first motor is configured to rotate to drive the first arm to rotate relative to the base;
> a first gravity compensation mechanism connected between the base and the first arm; and
> a control device coupled with the first motor, wherein the control device is configured to:

>> responding to the first arm moving to a first position, and determine a first moment that needs to be compensated when the first arm is at the first position;
>> determine a second sub force moment that needs to be compensated by the first motor, based on the first force moment and a first sub force moment compensated by the first gravity compensation mechanism; and
>> drive the first motor to output the second sub force moment.

Wherein, the input device further includes a first sensor, the first sensor is coupled with the control device and configured to sense position change of the first arm. Responding to the first arm moving to the first position includes: responding to an information as to the first position sensed by the first sensor.

Wherein, the input device further includes a first force sensor, the first force sensor is coupled with the control device and configured to sense a first sub force moment actually compensated by the first gravity compensation mechanism, and determining the second sub force moment that needs to be compensated by the first motor including:

> obtaining the first sub force moment through the first force sensor; and
> determining the second sub force moment through following formula, in which the second sub force moment is equal to the difference between the first force moment and the first sub force moment.

Wherein, the input device further includes determining the second sub force moment based on a compensation ratio of the first motor and the first gravity compensation mechanism.

The present application further provides a surgical robot, including any input device mentioned above.

The present application sets the first transmission mechanism between the first motor and the arm turntable, and deceleration is achieved by the first transmission mechanism. As a result, the rotational speed of the output shaft of the first motor is greater than that of the arm turntable. Compared to the solution where the output shaft of the first motor is directly fixed to the arm turntable, the present embodiment uses a smaller motor to achieve the rotation of the arm turntable, thereby reducing the size of the whole input device, achieving miniaturization of the input device 10, and improving the flexibility for operating the input device.

BRIEF DESCRIPTION OF THE DRAWINGS

> FIG. 1 shows a slave operating device of a surgical robot according to an embodiment of the present application;
> FIG. 2 shows a master console of the surgical robot according to an embodiment of the present application;
> FIG. 3 is a diagrammatic view of an input device of the master console shown in FIG. 2 in an embodiment;
> FIG. 4 is a diagrammatic view of portions of the input device shown in FIG. 3;
> FIG. 5 is a diagrammatic view of the portions shown in FIG. 4 from another angle;
> FIG. 6 is a diagrammatic view of a base shown in FIG. 5;
> FIG. 7 is a diagrammatic view of the structure shown in FIG. 5 from another angle;
> FIG. 8A is a diagrammatic view of the structure shown in FIG. 5 from another angle;

FIG. 8B is a diagrammatic view of portions of a first gravity compensation mechanism in another embodiment;

FIG. 9 is a cross-sectional view of the structure shown in FIG. 8A;

FIG. 10A is an enlarged diagrammatic view of region I of the structure shown in FIG. 9;

FIG. 10B is a diagrammatic view of the structure shown in FIG. 8A in another embodiment;

FIG. 11 is a diagrammatic view of the structure shown in FIG. 5 from another angle;

FIG. 12 is a cross-sectional view of the structure shown in FIG. 11 from another angle;

FIG. 13 is an enlarged diagrammatic view of region II of the structure shown in FIG. 12;

FIG. 14 is a flowchart of a control method for an input device;

FIG. 15 is another flowchart of a control method for an input device.

## DETAILED DESCRIPTION

Implementations of the present disclosure will now be described, by way of embodiments, with reference to the above figures. The embodiments are obviously a portion but not all of the embodiments of the present application. Based on the embodiments of the present application, other embodiments obtained by ordinary skill in the art without creative work will still fall within the scope of protection of the present application.

It should be noted that the directional indications (such as up, down, left, right, front, back) in the embodiments of the present invention are only used to explain the relative position relationships or motions between different components in a specific posture (as shown in the attached figures). If the specific posture changes, the directional indications will also change accordingly.

In the description of the present invention, it should be noted that unless otherwise specified, the terms "couple" and "connect" should be broadly understood. For example, the term "connect" may mean fixedly connect, detachably connect, or integrally connect. The term "connect" may also be understood as mechanically connect, electrically connect, or communicatively connect. The term "connect" may also be understood as directly connect or indirectly connect through an intermediate medium. The term "connect" may also be understood as internally connect two components or interact between the two components. For one having ordinary skill in the art, the specific meanings of the above terms in the present application may be understood based on specific circumstances.

As used herein, the terms "distal end" and "proximal end" are common directional terms in the art of interventional medical devices, wherein he "distal end" refers to an end far away from the operator during the surgical procedure, and the "proximal end" refers to an end close to the operator during the surgical procedure.

In addition, the terms "first" and "second" are mainly used for describing, which are not intended to indicate or imply the relative importance and quantity of the indicated features. Thus, the feature limited by the term "first" r "second" may mean that at least of the related feature is included.

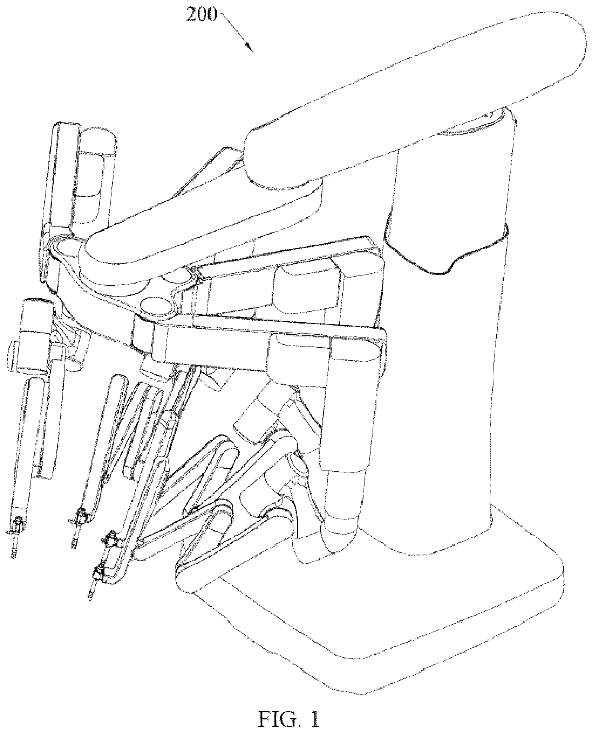

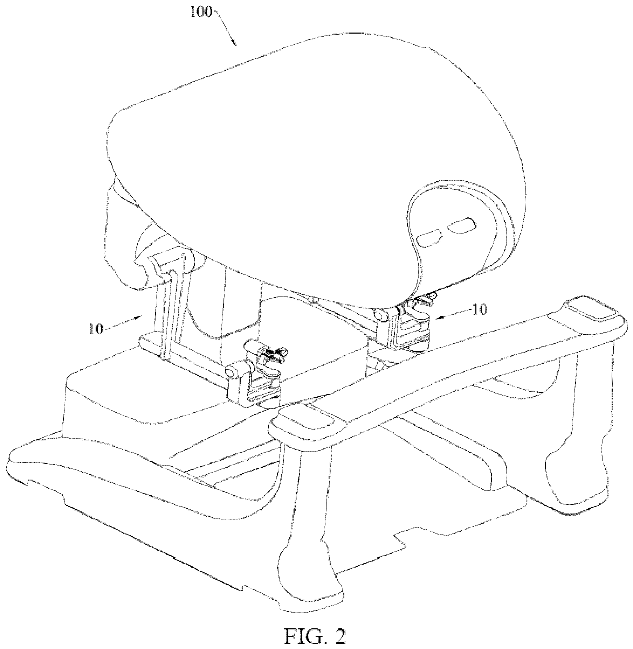

Referring to FIGS. 1 and 2, wherein FIG. 1 shows a slave operating device of a surgical robot according to an embodiment of the present application, and FIG. 2 shows a master console of the surgical robot according to an embodiment of the present application.

The surgical robot may include the master console 100 and the slave operating device 200, which are communicatedly connected to each other. The master console 100 sends control commands to the slave operating device 200 according to operations of a doctor, and the control commands are used to control the slave operating device 200. The slave operating device 200 performs corresponding surgical operations in response to the control commands from the master console 100. Of course, in other embodiments, the master console and slave operating devices may also be integrated into an integrated machine.

The master console 100 and the slave operating device 200 may be placed in a surgery room or different rooms, and the master console 100 and the slave operating device 200 may be far from each other. For example, when the master console 100 and the slave operating device 200 are located in different cities, the master console 100 and the slave operating device 200 may transmit data therebetween through wired or wireless manners. For example, when the master console 100 and the slave operating device 200 are located in the same surgery room, the master console 100 and the slave operating device 200 may transmit data therebetween through wired manner. For example, when the master console 100 and the slave operating device 200 are located in different cities, the master console 100 and the slave operating device 200 may remotely transmit data therebetween through wireless manner.

The master console 100 has at least one input device 10 and a display device. The doctor sends control commands to the slave operating device 200 through the input device 10, such that the slave operating device 200 performs the corresponding operations based on the control commands of the doctor by operating the input device 10, and the doctor further observes the surgical area on the display device. In one embodiment, there are two input devices 10, and the doctor operates one input device 10 with one hand. Of course, in other embodiments, the quantity of the input device(s) 10 may also be one or more.

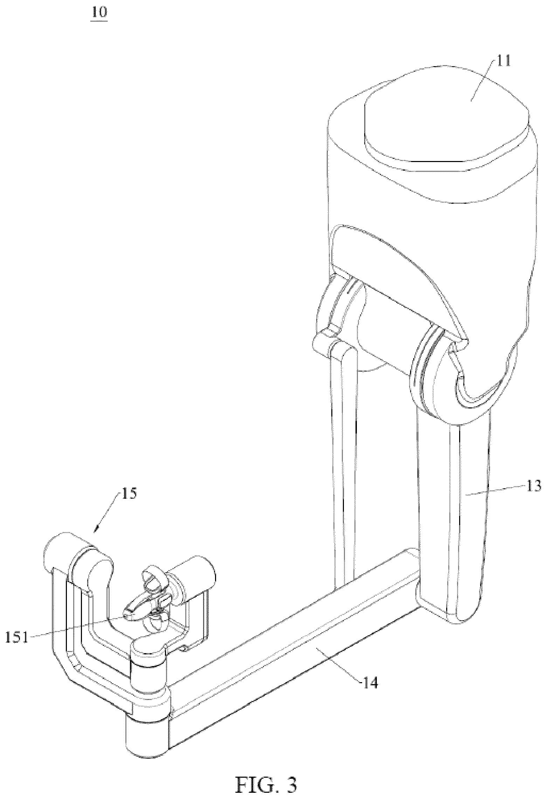

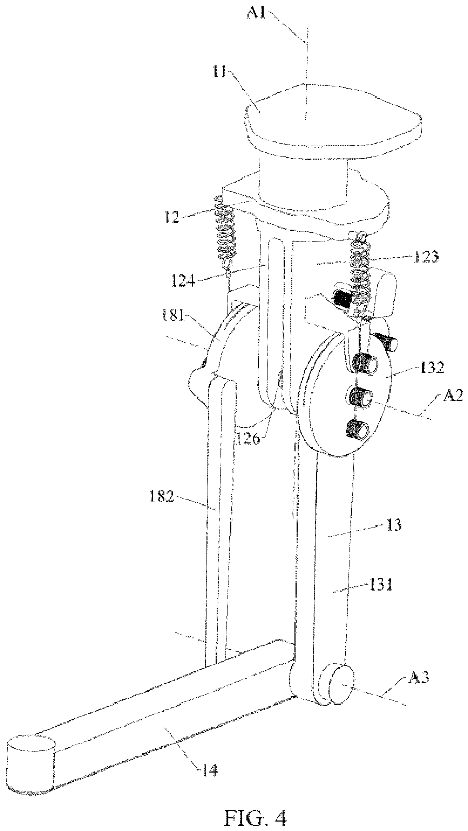

Referring to FIGS. 3 and 4, wherein FIG. 3 is a diagrammatic view of the input device 10 of the master console shown in FIG. 2 in some embodiments, and FIG. 4 is a diagrammatic view of portions of the input device shown in FIG. 3.

The input device 10 may include a connecting member 11, a base 12, an operating assembly 15, and at least one driving arm. The connecting member 11, the base 12, the at least one driving arm, and the operating assembly 15 are sequentially and rotatably connected to each other. An end of the connecting member 11 away from base 12 is connected to a relevant component of the master console 100. The operating assembly 15 is used to receive the operations from the operator. The operating assembly 15 may include a handle 151. The operator grasps the handle 151 to perform the operations to the input device 10, thereby sending the control commands to the slave operating device 200.

The quantity of the at least one driving arm may be set according to actual needs. For example, when the motion to be executed is relatively simple, only one driving arm may be needed. One end of the driving arm is connected to the base 12, and another end of the driving arm is connected to the operating assembly 15. When the motion to be executed is complex, two or more driving arms may be selected. The more driving arms, the higher the degree of freedom, which may execute operations with higher complexity.

The embodiment takes two driving arms as an example. The two driving arms include a first arm 13 and a second arm 14. The second arm 14 is rotatably connected to the first arm 13. One end of the first arm 13 away from the second arm 14 is rotatably connected to the base 12. One end of the second arm 14 away from the first arm 13 is rotatably connected to the operating assembly 15. The rotations of the first arm 13 and the second arm 14 allow the operating assembly 15 to move in at least two degrees of freedom.

In one embodiment, the base 12 rotates about a first axis A1 relative to the connecting member 11, the first arm 13 rotates about a second axis A2 relative to the base 12, and the second arm 14 rotates about a third axis A3 relative to the first arm 13. The first axis A1 is perpendicular to the second axis A2 and the third axis A3, and the second axis A2 is parallel to the third axis A3, such that the rotations of the base 12, the first arm 13, and the second arm 14 may allow the operating assembly 15 to move in a three-dimensional space.

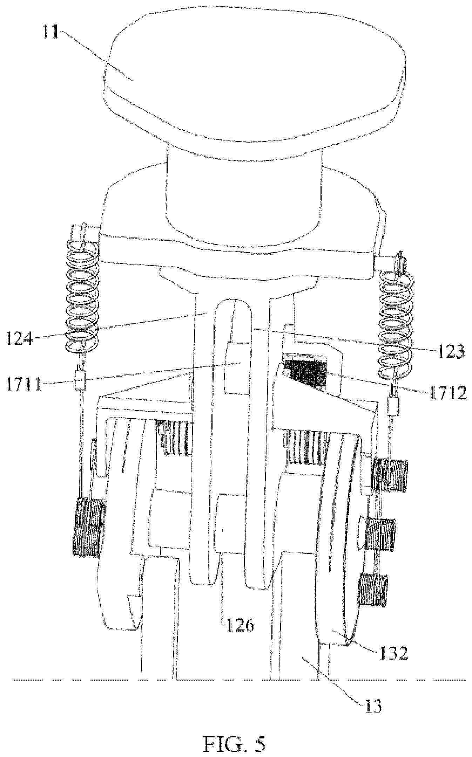

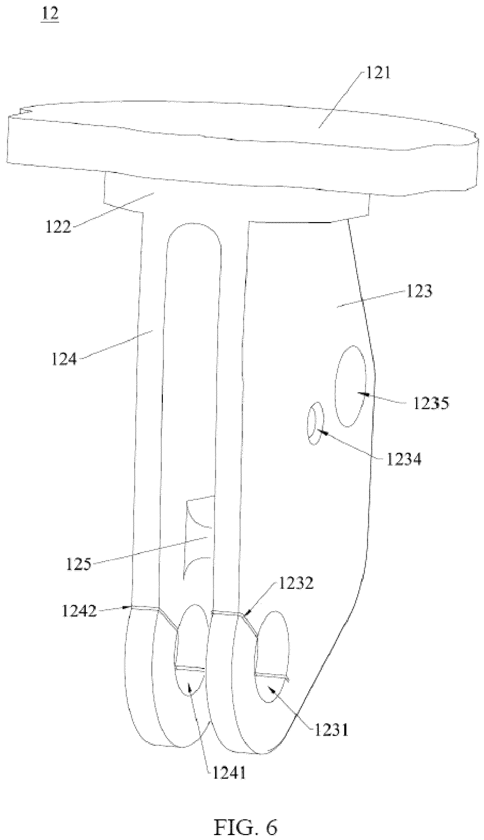

Referring to FIGS. 5 and 6, wherein FIG. 5 is a diagrammatic view of portions of the structure shown in FIG. 4 from another angle, and FIG. 6 is a diagrammatic view of the base shown in FIG. 5.

The base 12 may include a main turntable 121, a main body 122, a first mounting plate 123, and a second mounting plate 124. The main turntable 121 is rotatably connected to the connecting member 11. The main body 122 is fixed to a side of the main turntable 121 away from the connecting member 11. The first mounting plate 123 and the second mounting plate 124 are connected to the main body 122 and opposite to each other. The first mounting plate 123 and the second mounting plate 124 are located at a side of the main body 122 away from the main turntable 121. The first arm 13 is rotatably connected to one end of the first mounting plate 123 away from the main body 122, and is located at a side of the first mounting plate 123 away from the second mounting plate 124. The second mounting plate 124 is used to connect other components.

It should be noted that since the first mounting plate 123 and the second mounting plate 124 are opposite to each other, a certain gap is defined between the first mounting plate 123 and the second mounting plate 124. Of course, in other embodiments, no gap is formed between the first mounting plate 123 and the second mounting plate 124, that is, the first mounting plate 123 and the second mounting plate 124 are integrated into a solid mounting plate. However, the solid mounting plate is heavier compared to the structure with the gap formed between the first mounting plate 123 and the second mounting plate 124, which is not conducive to lightweight requirement of the input device 10 for the operator to operate easily.

In one embodiment, the main body 122, the first mounting plate 123, and the second mounting plate 124 are integrated into a single structure to increase the connection strength, thereby avoiding the need to assemble the three components together and improving the production efficiency. Of course, in one scenario of other embodiments, the first mounting plate 123, the second mounting plate 124, and the main body 122 may also be connected together by screwing, welding, or other connection methods. In another scenario of other embodiments, the main body 122, the first mounting plate 123, the second mounting plate 124, and the main turntable 121 may also be an integrated structure.

In one embodiment, as shown in FIGS. 5 and 6, a reinforcement member 125 is provided between the first mounting plate 123 and the second mounting plate 124. The reinforcement member 125 is located at one end of the two mounting plates near the first arm 13, which ensures that the first mounting plate 123 and the second mounting plate 124 will not deviate in the extension direction of the first axis A1 (shown in FIG. 4), and enhances the connection strength of the whole base 12. Of course, in other embodiments, no reinforcement member may be provided between the first mounting plate 123 and the second mounting plate 124.

The input device 10 may further include a fixing shaft 126. The fixing shaft 126 extends through and is fixed to the first mounting plate 123 and the second mounting plate 124. Two ends of the fixing shaft 126 are rotatably connected to the first arm 13 and other components, respectively. That is, in the embodiment, two components are rotatably connected to the first mounting plate 123 and the second mounting plate 124, respectively, through the fixing shaft 126, thereby simplifying the structure of the input device 10, reducing the weight of the input device 10, and improving the operation experience of the operator. Meanwhile, since the fixing shaft 126 extends through the first mounting plate 123 and the second mounting plate 124, the connection strength among the fixing shaft 126 and the two mounting plates is increased.

Of course, in other embodiments, the first arm 13 may also be directly and rotatably connected to a surface of the first mounting plate 123 away from the second mounting plate 124, and other components may also be directly and rotatably connected to a surface of the second mounting plate 124 away from the first mounting plate 123.

For example, as shown in FIGS. 5 and 6, the end of the first mounting plate 123 away from the main body 122 defines a first fixing hole 1231, and the first fixing hole 1231 extends through two opposite surfaces of the first mounting plate 123. The end of the second mounting plate 124 away from the main body 122 defines a second fixing hole 1241, and the second fixing hole 1241 extends through two opposite surfaces of the second mounting plate 124. The first fixing hole 1231 and the second fixing hole 1241 are coaxial to each other. The fixing shaft 126 sequentially extends through the first fixing hole 1231 and the second fixing hole 1241. The fixing shaft 126 is fixed to the first mounting plate 123 and the second mounting plate 124 through the first fixing hole 1231 and the second fixing hole 1241.

In one embodiment, the first mounting plate 123 defines a first notch 1232. The first notch 1232 is connected to the inner and outer walls of the first fixing hole 1231. That is, the walls of the first fixing hole 1231 are not enclosed. The second mounting plate 124 defines a second notch 1242. The second notch 1242 is connected to the inner and outer walls of the second fixing hole 1241. That is, the walls of the second fixing hole 1241 are not enclosed. After the fixing shaft 126 is located in the first fixing hole 1231 and the second fixing hole 1241, a screw is rotatably connected to the walls at two sides of each notch, thereby reducing the width of each of the first notch 1232 and the second notch 1242. Thus, the walls of the first fixing hole 1231 and the second fixing hole 1241 are tightly attached and fixed to the fixing shaft 126, thereby fixing the fixing shaft 126 to the first mounting plate 123 and the second mounting plate 124. Of course, in other embodiments, the fixing shaft 126 may also be fixed to the first mounting plate 123 and the second mounting plate 124 through other fixing methods such as screwing, snap-fitting, and welding.

Referring to FIGS. 4 and 5, the first arm 13 may include an arm body 131 and an arm turntable 132 fixed to one end of the arm body 131. In one embodiment, the arm body 131 is detachably connected to the arm turntable 132. One side of the arm body 131 away from the arm turntable 132 is rotatably connected to the end of the fixing shaft 126 extending through the first mounting plate 123. That is, the arm turntable 132 is mounted on the first mounting plate 123 through the arm body 131, and the end of the arm body 131 away from the arm turntable 132 is rotatably connected to the second arm 14. Of course, in other embodiments, the arm body 131 and the arm turntable 132 may also be an integrated structure. Alternatively, the arm turntable 132 may also be directly mounted on the first mounting plate 123.

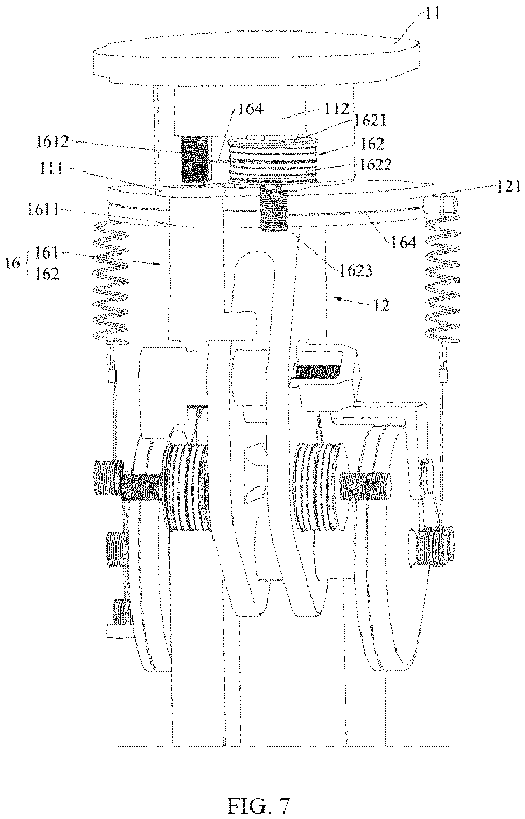

Referring to FIG. 7, wherein FIG. 7 is a diagrammatic view of the structure shown in FIG. 5 from another angle.

The input device 10 may further include a main driving device 16. The main driving device 16 drives the base 12 to rotate relative to the connecting member 11 about the first axis A1 (FIG. 4).

In some embodiments, the main driving device 16 may include a main motor 161, a main transmission mechanism, and a main sensor. An extension line in the length direction of the main motor 161 is perpendicular to the rotation axis of the arm turntable 132, thereby ensuring the transverse miniaturization of the input device.

The main transmission mechanism includes a main adjusting member 162. An output shaft of the main motor 161 is rotatably connected to an input portion of the main adjusting member 162, and an output portion of the main adjusting member 162 is rotatably connected to the main turntable 121 of the base 12. When the main motor 161 rotates, the main motor 161 drives the main adjusting member 162 to rotate, such that the base 12 is driven to rotate. The main sensor senses any position change of the base 12.

In one embodiment, the main motor 161 may include a main portion 1611 and an output shaft 1612 connected to the main portion 1611. The connecting member 11 includes a first connecting structure 111 and a second connecting structure 112 spaced from each other. The main portion 1611 is mounted on the first connecting structure 111, and one end of the output shaft 1612 away from the main portion 1611 is rotatably connected to the second connecting structure 112. That is, each of two ends of the main motor 161 is connected to the connecting member 11 through one connecting structure, such that the main motor 161 is stably mounted on the connecting member 11, and the output shaft 1612 stably outputs during the rotation. Of course, in other embodiments, the end of the output shaft 1612 away from the main portion 1611 may also be directly and rotatably connected to the connecting member 11.

The main adjusting member 162 may include a fixing portion 1621, an input portion 1622, and an output portion 1623. The input portion 1622 is fixed to the output portion 1623. Each of the input portion 1622 and the output portion 1623 is rotatably connected to the fixing portion 1621. The fixing portion 1621 is fixed to the second connecting structure 112, and is connected to the connecting member 11 through the second connecting structure 112. Of course, in other embodiments, the fixing portion 1621 may also be directly fixed to the connecting member 11.

In one embodiment, the output shaft 1612 of the main motor 161 is rotatably connected to the input portion 1622 of the main adjusting member 162 through a driving rope 164. The output portion 1623 of the main adjusting member 162 is also rotatably connected to the main turntable 121 through the driving rope 164. The driving rope 164 may include a steel wire rope for example, which has a high safety factor during load-bearing and is safe and reliable to use. The steel wire rope also has a high tensile strength, fatigue strength, impact toughness, wear resistance, earthquake resistance, and good operational stability.

Of course, in other implementations, the output shaft 1612 and the input portion 1622 may also be rotatably connected to each other through a conveyor belt or gear wheel. Similarly, the output portion 1623 and the main turntable 121 may also be rotatably connected through another conveyor belt or gear wheel.

In one embodiment, a radius of the input portion 1622 of the main adjusting member 162 is greater than a radius of the output shaft 1612, thereby achieving a first stage deceleration. A radius of the main turntable 121 is greater than a radius of the output portion 1623 of the main adjusting member 162, thereby achieving a second stage deceleration.

That is, by limiting the radius of the input portion 1622 to be greater than the radius of the output shaft 1612 and limiting the radius of the main turntable 121 to be greater than the radius of the output portion 1623, two-stage deceleration is achieved. Compared to the solution where the output shaft 1612 of the main motor 161 is directly fixed to the main turntable 121, the preset embodiment uses a smaller motor to achieve the rotation of the main turntable 121, thereby reducing the size of the whole input device 10 and realizing the miniaturization of the input device 10. At the same time, the connection between the output shaft 1612 and the main turntable 121 is achieved through the adjusting member, thereby allowing the position of the main motor 161 to be more flexible to a certain extent and further realizing the miniaturization of the input device 10.

Of course, in one scenario of other embodiments, the radius of the input portion 1622 of the main adjusting member 162 may also be equal to the radius of the output shaft 1612, and the radius of the output portion 1623 may be smaller than the radius of the main turntable 121.

In another scenario of other embodiments, the radius of the input portion 1622 of the main adjusting member 162 may also be greater than the radius of the output shaft 1612, and the radius of the output portion 1623 may also be equal to the radius of the main turntable 121.

In yet another scenario of other embodiments, the main driving device 16 may only include the main motor 161 and the main sensor. That is, the main driving device 16 in the embodiment may not include the main adjusting member. The output shaft 1612 of the main motor 161 may be rotatably connected to the main turntable 121 through a driving rope. The driving rope may include a steel wire rope for example.

For example, the radius of the main turntable 121 is greater than the radius of the output shaft 1612. By limiting the radius of the main turntable 121 to be greater than the radius of the output shaft 1612, deceleration may be achieved. Compared to the solution where the output shaft 1612 of the main motor 161 is directly fixed to the main turntable 121, the present embodiment uses a smaller motor to achieve the rotation of the main turntable 121, which is conducive to the miniaturization of the input device 10. Of course, in other implementations, the output shaft 1612 and the main turntable 121 may also be rotatably connected to each other through a conveyor belt or gear wheel.

In other embodiments, the output shaft of the main motor may also be directly fixed to the main turntable.

The main sensor is coupled to the main turntable 121, and senses the position change of the base 12 when the base 12 rotates about the first axis A1. In some embodiments, the main sensor is a rotation sensor, such as a single-turn absolute encoder, a multi-turn absolute encoder, or other sensors that sense rotation.

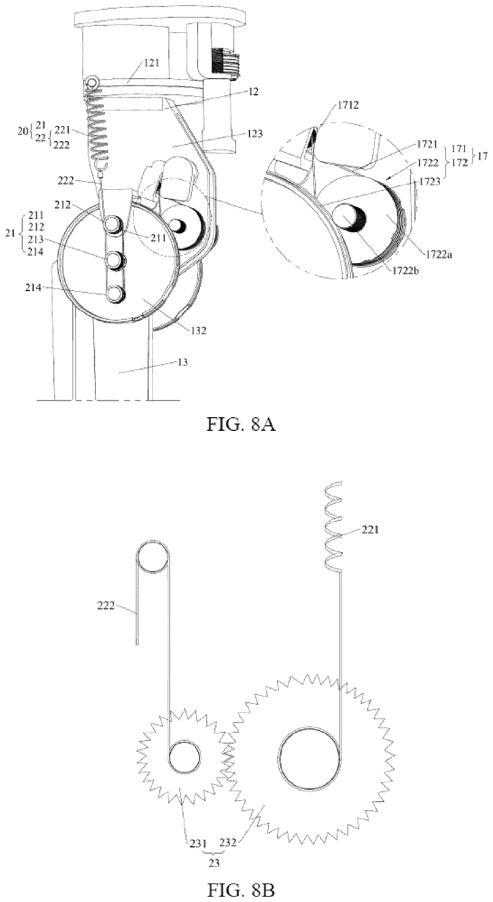

Referring to FIGS. 5 and 8A, wherein FIG. 8A is a diagrammatic view of the structure shown in FIG. 5 from another angle.

The input device 10 may further include a first driving device 17. The first driving device 17 drives the first arm 13 to rotate about the second axis A2 (shown in FIG. 4) relative to the base 12.

In some embodiments, the first driving device 17 may include a first motor 171, a first transmission mechanism 172, and a first sensor. The arm turntable 132 is rotatably connected to an output shaft 1712 of the first motor 171 through the first transmission mechanism 172. When the output shaft 1712 rotates, the output shaft 1712 drives the arm turntable 132 to rotate, thereby causing the first arm 13 to rotate relative to the base 12. A rotational speed of the output shaft 1712 of the first motor 171 is greater than that of the arm turntable 132. The first sensor senses any position change of the first arm 13.

The embodiment sets the first transmission mechanism 172 between the first motor 171 and the arm turntable 132, and deceleration is achieved by the first transmission mechanism 172. As a result, the rotational speed of the output shaft 1712 of the first motor 171 is greater than that of the arm turntable 132. Compared to the solution where the output shaft 1712 of the first motor 171 is directly fixed to the arm turntable 132, the present embodiment uses a smaller motor to achieve the rotation of the arm turntable 132, thereby reducing the size of the whole input device 10, achieving miniaturization of the input device 10, and improving the flexibility for operating the input device 10.

At the same time, the rotation connection between the output shaft 1712 of the first motor 171 and the arm turntable 132 is realized by the first transmission mechanism 172, which allows the position of the first motor 171 to be more flexible and achieving the miniaturization of the input device 10.

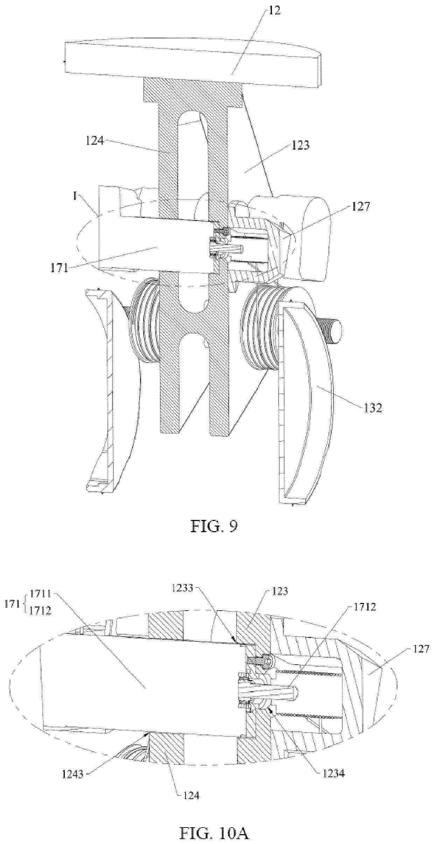

Referring to FIGS. 8A, 9, and 10A, wherein FIG. 9 is a cross-sectional view of the structure shown in FIG. 8A, and FIG. 10A is an enlarged diagrammatic view of the region I shown in FIG. 9.

The first motor 171 is mounted on the base 12. The first motor 171 may include a motor body 1711 and the output shaft 1712 connected to the motor body 1711. The motor body 1711 of the first motor 171 is mounted on the first mounting plate 123. The output shaft 1712 of the first motor 171 is spaced from the arm turntable 132. An end of the first motor 171 near the second mounting plate 124 (i.e., the end away from the output shaft 1712) extends through the second mounting plate 124, such that the first motor 171 overlaps with the first mounting plate 123, the second mounting plate 124, and the space therebetween in the length direction, which is conducive to achieving the transverse miniaturization of the input device 10. Of course, in other embodiments, the motor body 1711 of the first motor 171 may also be mounted on the first mounting plate 123 without extending through the second mounting plate 124.

In some embodiments, the first mounting plate 123 may include a first fixing groove 1233 and a first avoidance hole 1234. The first avoidance hole 1234 is located in a middle area of a bottom wall of the first fixing groove 1233, and extends through the bottom wall of the first fixing groove 1233 and the surface of the first mounting plate 123 away from the second mounting. The second mounting plate 124 may include a first through hole 1243. The motor body 1711 extends through the first through hole 1243 and is mounted in the first fixing groove 1233. The output shaft 1712 of the first motor 171 extends through the first avoidance hole 1234 of the first mounting plate 123, and is spaced from the arm turntable 132. Of course, in other embodiments, the manner that the first motor 171 being mounted to the first mounting plate 123 is limited to the above description.

In one embodiment, the first mounting plate 123 includes a first fixing member 127. The end of the output shaft 1712 of the first motor 171 away from the motor body 1711 is rotatably connected to the first fixing member 127, such that the first motor 171 is stably mounted to the base 12, and the output shaft 1712 stably outputs during the rotation.

The first transmission mechanism 172 may include a first transmission member 1721, a first adjusting member 1722, and a second transmission member 1723. The first transmission member 1721 is rotatably connected between the output shaft 1712 and an input portion 1722a of the first adjusting member 1722. The second transmission member 1723 is connected between an output portion 1722b of the first adjusting member 1722 and the arm turntable 132. The specific structure of the first adjusting member 1722 in the embodiment is the same as that of the main adjusting member 162, which will not be repeated. The first adjusting member 1722 is mounted on the first mounting plate 123 and located between the first motor 171 and the arm turntable 132.

In one embodiment, each of the first transmission member 1721 and the second transmission member 1723 includes a driving rope. The driving rope may include a steel wire rope for example. The steel wire rope has a high safety factor during load-bearing, and is safe and reliable to use. The steel wire rope also has high tensile strength, fatigue strength, impact toughness, wear resistance, earthquake resistance, and good operational stability. Of course, the first transmission member 1721 and the second transmission member 1723 may also be other transmission members such as conveyor belts or gear wheel.

For example, the first transmission member 1721 may include a first drive rope and a second drive rope. The first drive rope and the second drive rope are arranged in a crossed manner. Two ends of the first driving rope are fixed clockwise around the output shaft 1712 and the input portion 1722a, respectively. Two ends of the second driving rope are fixed counterclockwise around the output shaft 1712 and the input portion 1722a, respectively. Of course, in other implementations, the two ends of the first driving rope may also be fixed counterclockwise around the output shaft 1712 and the input portion 1722a, respectively. The two ends of the second driving rope may also be fixed clockwise around the output shaft 1712 and the input portion 1722a, respectively. For example, the second transmission member 1723 may include a third driving rope and a fourth driving rope. The third drive rope and the fourth drive rope are arranged in a crossed manner. Two ends of the third driving rope are fixed clockwise around the output portion 1722b and the arm turntable 132, respectively. Two ends of the fourth drive rope are fixed counterclockwise around the output portion 1722b and the arm turntable 132, respectively. Of course, in other implementations, the two ends of the third drive rope may also be fixed counterclockwise around the output portion 1722b and the arm turntable 132, respectively. The two ends of the fourth drive rope may also be fixed clockwise around the output portion 1722b and the arm turntable 132, respectively.

Therefore, whether the output shaft 1712 rotates clockwise or counterclockwise, the output shaft 1712 is able to drive the arm turntable 132 to rotate clockwise or counterclockwise.

Of course, in other embodiments, the rotation connection between the output shaft 1712 and the input portion 1722a may also be achieved through a conveyor belt or gear wheel. Similarly, the output portion 1722b may also be rotatably connected to the arm turntable 132 through a conveyor belt or gear wheel.

In one embodiment, a radius of the input portion 1722a of the first adjusting member 1722 is greater than a radius of the output shaft 1712, thereby achieving a first stage deceleration. A radius of the arm turntable 132 is greater than a radius of the output portion 1722b of the first adjusting member 1722, thereby achieving a second stage deceleration.

That is, by limiting the radius of the input portion 1722a to be greater than the radius of the output shaft 1712 and limiting the radius of the arm turntable 132 to be greater than the radius of the output portion 1722b, two-stage deceleration is achieved. Compared to the solution where the output shaft 1712 of the first motor 171 is directly fixed to the arm turntable 132, the present embodiment uses a smaller motor to achieve the rotation of the arm turntable 132, thereby reducing the volume of the whole input device 10 and realizing the miniaturization of the input device 10.

At the same time, since the output shaft 1712 and the arm turntable 132 are connected to each other through the adjusting member, the position of the first motor 171 is more flexible, thereby further achieving the miniaturization of the input device 10.

Of course, in one scenario of other embodiments, the radius of the input portion 1722a of the first adjusting member 1722 may also be equal to the radius of the output shaft 1712, and the radius of the output portion 1722b may be smaller than the radius of the arm turntable 132.

In another scenario of other embodiments, the radius of the input portion 1722a of the first adjusting member 1722 is greater than the radius of the output shaft 1712, and the radius of the output portion 1722b is equal to the radius of the arm turntable 132.

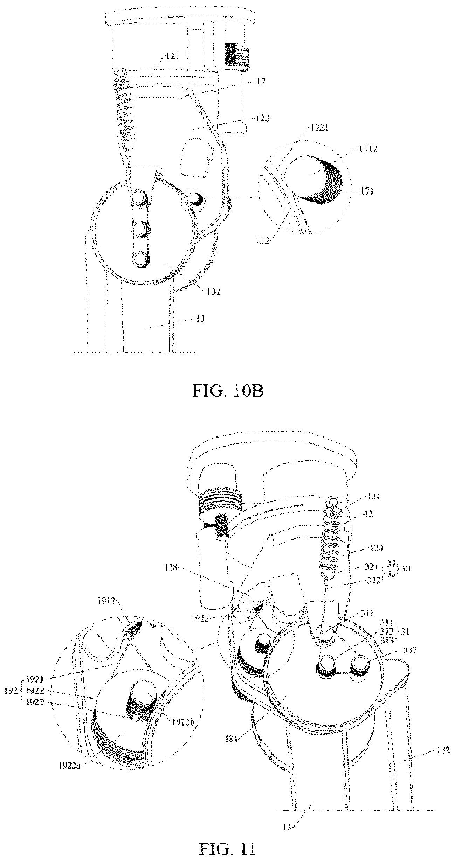

Referring to FIG. 10B, which is a diagrammatic view of the structure shown in FIG. 8A in another embodiment.

In another embodiment, the first transmission mechanism may only include the first transmission member 1721, that is, the first transmission mechanism 172 may not include the first adjusting member 1722 and the second transmission member 1723. The first transmission member 1721 is connected between the arm turntable 132 and the output shaft 1712 of the first motor 171, that is, the arm turntable 132 and the output shaft 1712 are rotatably connected to each other through the first transmission member 1721. The first transmission member 1721 may include a steel wire rope for example. The manner in which the first transmission member 1721 is rotatably connected between the arm turntable 132 and the output shaft 1712 is the same as the manner in which the first transmission member 1721 is rotatably connected between the output shaft and the input portion in FIG. 8A, which will not be repeated.

The radius of the arm turntable 132 is greater than the radius of the output shaft 1712 of the first motor 171. By limiting the radius of the arm turntable 132 to be greater than the radius of the output shaft 1712, deceleration is achieved. Compared to the solution where the output shaft 1712 of the first motor 171 is directly fixed to the arm turntable 132, the present embodiment uses a smaller motor to achieve the rotation of the arm turntable 132, which is conducive to the miniaturization of the input device 10. Of course, in other implementations, the rotation connection between the output shaft 1712 and the arm turntable 132 may also be achieved through a transmission belt or gear wheel.

In other embodiments, the output shaft of the first motor may also be directly fixed to the first arm.

The first sensor is coupled to the arm turntable 132, and senses the position change of the first arm 13 when the first arm 13 rotates about the second axis A2. In some embodiments, the first sensor is a rotation sensor, such as a single-turn absolute encoder, a multi-turn absolute encoder, or other sensors that sense rotation.

Referring to FIG. 4, the input device 10 may further include a transmission turntable 181 and a transmission arm 182. The transmission turntable 181 is rotatably connected to the base 12. The transmission arm 182 is rotatably connected between the transmission turntable 181 and the second arm 14. When the transmission turntable 181 rotates, the transmission turntable 181 drives the transmission arm 182 to move, thereby driving the second arm 14 to rotate relative to the first arm 13.

For example, the transmission turntable 181 is rotatably connected to the end of the fixing shaft 126 extending through the second mounting plate 124. The transmission turntable 181, the transmission arm 182, the first arm 13, and the second arm 14 form a parallelogram mechanism. It is understandable that in the embodiment, the transmission turntable 181 and the arm turntable 132 are respectively located on two opposite sides of the base 12, thereby ensuring the balance of the base 12 under force.

Of course, in other embodiments, the input device 10 may not include the transmission turntable 181 and the transmission arm 182. The input device 10 may be driven by a wire or other driving mechanisms to rotate the second arm 14 relative to the first arm 13.

Referring to FIG. 11, which is a diagrammatic view of the structure shown in FIG. 5 from another angle.

The input device 10 may further include a second driving device 19. The second driving device 19 drives the transmission turntable 181 to rotate about the second axis A2 (shown in FIG. 4) relative to the base 12, thereby driving the transmission arm 182 to linearly move, and then driving the second arm 14 to rotate about the third axis A3 (shown in FIG. 3) relative to the first arm 13.

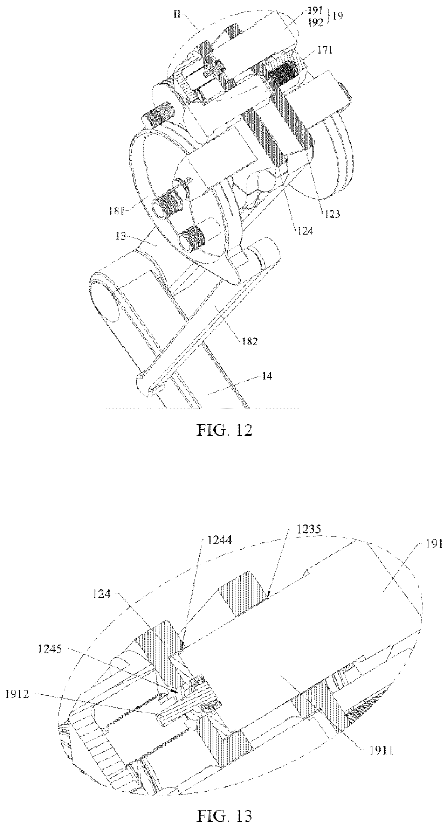

In some embodiments, the second driving device 19 may include a second motor 191 (as shown in FIG. 12), a second transmission mechanism 192, and a second sensor. An output shaft 1912 of the second motor 191 is rotatably connected to the transmission turntable 181 through the second transmission mechanism 192. The second motor 191 drives the transmission turntable 181 to rotate through the second transmission mechanism 192, thereby driving the second arm 14 to rotate. Of course, in other embodiments, the output shaft 1912 of the second motor 191 may also be rotatably connected to the second arm 14 through the second transmission mechanism 192. The second sensor senses any position change of the second arm 14.

The embodiment sets the second transmission mechanism 192 between the second motor 191 and the transmission turntable 181, thereby achieving deceleration. Compared to the solution where the output shaft 1912 of the second motor 191 is directly fixed to the transmission turntable 181, the present embodiment uses a smaller motor to rotate the transmission turntable 181, thereby reducing the size of the whole input device 10 and achieving miniaturization of the input device 10.

At the same time, since the output shaft 1912 of the second motor 191 and the transmission turntable 181 are rotatably connected to each other through the second transmission mechanism 192, the position of the second motor 191 more flexible, thereby further achieving the miniaturization of the input device 10.

Referring to FIGS. 11, 12, and 13, wherein FIG. 12 is a cross-sectional view of the structure shown in FIG. 11 from another angle, and FIG. 13 is an enlarged diagrammatic view of the region II of the structure shown in FIG. 12.

The second motor 191 is mounted on the base 12. The second motor 191 may include a motor body 1911 and an output shaft 1912 connected to the motor body 1911. The motor body 1911 of the second motor 191 is mounted on the second mounting plate 124. The output shaft 1912 of the second motor 191 is spaced from the transmission turntable 181. The end of the second motor 191 near the first mounting plate 123 (i.e., the end away from the output shaft 1912) extends through the first mounting plate 123, such that the second motor 191 overlaps with the space between the first mounting plate 123 and the second mounting plate 124 in the length direction of the second motor 191, which is conducive to achieving the transverse miniaturization of the input device 10.

Of course, in other embodiments, the motor body 1911 of the second motor 191 may also be mounted on the second mounting plate 124 without extending through the first mounting plate 123.

In one embodiment, each of the second motor 191 and the first motor 171 extends through the first mounting plate 123 and the second mounting plate 124, and the second motor 191 and the first motor 171 are parallel to each other. For example, the extension line of each of the first motor 171 and the second motor 191 in the length direction is parallel to the rotation axis of the arm turntable 132. Compared to the solution where the first motor 171 and the second motor 191 are side by side and spaced from each other in the axial direction thereof, the present embodiment reduces the transverse size of the input device 10, which is conducive to the miniaturization of the input device 10.

In some embodiments, the second mounting plate 124 may include a second fixing groove 1244 and a second avoidance hole 1245. The second avoidance hole 1245 is located in a middle area of a bottom wall of the second fixing groove 1244, and extends through the bottom wall of the second fixing groove 1244 and the surface of the second mounting plate 124 away from the first mounting. The first mounting plate 123 may include a second through hole 1235. The motor body 1911 extends through the second through hole 1235 and is mounted in the second fixing groove 1244. The output shaft 1912 of the second motor 191 extends through the second avoidance hole 1245 of the second mounting plate 124, and is spaced from the transmission turntable 181. Of course, in other embodiments, the manner in which the second motor 191 is mounted to the second mounting plate 124 is limited to the above description.

In one embodiment, the second mounting plate 124 may include a second fixing member 128. One end of the output shaft 1912 of the second motor 191 away from the motor body 1911 is rotatably connected to the second fixing member 128, such that the second motor 191 is stably mounted to the base 12, and the output shaft 1912 stably outputs during the rotation.

As shown in FIG. 11, the second transmission mechanism 192 may include a third transmission member 1921, a second adjusting member 1922, and a fourth transmission member 1923. The third transmission member 1921 is rotatably connected between the output shaft 1912 and the input portion 1922a of the second adjusting member 1922. The fourth transmission member 1923 is connected between the output portion 1922b of the second adjusting member 1922 and the transmission turntable 181.

The specific structure of the second adjusting member 1922 in the embodiment is the same as that of the main adjusting member 162, which will not be repeated. The second adjusting member 1922 is mounted on the second mounting plate 124 and located between the second motor 191 and the transmission turntable 181.

In one embodiment, each of the third transmission member 1921 and the fourth transmission member 1923 includes a driving rope. The driving rope may be a steel wire rope for example. The steel wire rope has a high safety factor during load-bearing, and is safe and reliable to use. The steel wire rope also has a high tensile strength, fatigue strength, impact toughness, wear resistance, earthquake resistance, and good operational stability.

It should be noted that the setting manner of the driving ropes for the third transmission member 1921 and the fourth transmission member 1923 may refer to the first transmission member and the second transmission member, which will not be repeated here.

Of course, in other embodiments, the output shaft 1912 and the input portion 1922a may also be rotatably connected to each other through a conveyor belt or gear wheel. Similarly, the output unit 1922b and the transmission turntable 181 may also be rotatably connected to each other through a transmission belt or gear wheel.

In one embodiment, a radius of the input portion 1922a of the second adjusting member 1922 is greater than a radius of the output shaft 1912, thereby achieving a first stage deceleration. A radius of the transmission turntable 181 is greater than a radius of the output portion 1922b of the second adjusting member 1922, thereby achieving a second stage deceleration.

That is, by limiting the radius of the input portion 1922a to be greater than the radius of the output shaft 1912 and limiting the radius of the transmission turntable 181 to be greater than the radius of the output portion 1922b, two-stage deceleration is achieved. Compared to the solution where the output shaft 1912 of the second motor 191 is directly fixed to the transmission turntable 181, the present embodiment uses a smaller motor to achieve the rotation of the transmission turntable 181, thereby reducing the size of the whole input device 10 and realizing the miniaturization of the input device 10. At the same time, since the output shaft 1912 and the transmission turntable 181 are connected to each other through the adjusting member, the position of the second motor 191 is more flexible, thereby further achieving the miniaturization of the input device 10.

Of course, in one scenario of other embodiments, the radius of the input portion 1922a of the second adjusting member 1922 may also be equal to the radius of the output shaft 1912, and the radius of the output portion 1922b may be smaller than the radius of the transmission turntable 181.

In another scenario in other embodiments, the radius of the input portion 1922a of the second adjusting member 1922 is greater than the radius of the output shaft 1912, and the radius of the output portion 1922b is equal to the radius of the drive turntable 181.

In other embodiments, the second transmission mechanism 192 may only include the third transmission member 1921. That is, the second transmission mechanism 192 may not include the second adjusting member 1922 and the fourth transmission member 1923. The third transmission member 1921 is connected between the transmission turntable 181 and the output shaft 1912 of the second motor 191, that is, the transmission turntable 181 and the output shaft 1912 are rotatably connected to each other through the third transmission member 1921. The third transmission member 1921 may be a steel wire rope for example.

The radius of the transmission turntable 181 is greater than the radius of the output shaft 1912 of the second motor 191. By limiting the radius of the transmission turntable 181 to be greater than the radius of the output shaft 1912, deceleration is achieved. Compared to the solution where the output shaft 1912 of the second motor 191 is directly fixed to the transmission turntable 181, the present embodiment uses a smaller motor to achieve the rotation of the transmission turntable 181, which is conducive to the miniaturization of the input device 10. Of course, in other implementations, the rotation connection between the output shaft 1912 and the transmission turntable 181 may also be achieved through a conveyor belt or gear wheel.

In other embodiments, the output shaft of the second motor may also be directly fixed to the transmission turntable.

The second sensor is coupled to the transmission turntable 181, and senses the position change of the second arm 14 when the second arm 14 rotates about the third axis A3. In some embodiments, the second sensor is a rotation sensor, such as a single-turn absolute encoder, a multi-turn absolute encoder, or other sensors that sense rotation.

Referring to FIG. 8A, the input device 10 may further include a first gravity compensation mechanism 20. The first gravity compensation mechanism 20 is connected between the base 12 and the first arm 13, and generates a force moment in a first degree of freedom, wherein the first degree of freedom is the rotation about the second axis A2, and the force moment may balance the gravity moment of the parallelogram mechanism. In some embodiments, the first gravity compensation mechanism 20 may include a first rotating mechanism 21 and a first elastic mechanism 22. The first rotating mechanism 21 may include a number of rotating portions. The rotating portions are distributed on the first mounting plate 123 of the base 12 and the first arm 13.

For ease of understanding, the first rotating mechanism 21 may include a first part of rotating portions and a second part of rotating portions. For example, the first part of rotating portions is located on the first mounting plate 123, and the second part of rotating portion is located on the arm turntable 132 of the first arm 13. The first elastic mechanism 22 is coupled between the main turntable 121 of the base 12 and the first rotating mechanism 21, and generates the force moment in the first degree of freedom that balances the gravity moment of the parallelogram mechanism. Thus, the operator is allowed to easily pull the input device 10 in the first degree of freedom.

In some embodiments, the first rotating mechanism 21 may include a first rotating portion 211, a second rotating portion 212, a third rotating portion 213, and a fourth rotating portion 214. The first rotating portion 211 is fixed to the first mounting plate 123 through an adapter. The second rotating portion 212 is coaxial with and rotates relative to the first rotating portion 211. The third rotating portion 213 and the fourth rotating portion 214 are rotatably connected to the first arm 13.

The rotation axis of the third rotating portion 213 coincides with the second axis A2. The rotation of the third rotating portion 213 is independent from the rotation of the first arm 13 about the second axis A2. The rotation axis of each of the second rotating portion 212 and the fourth rotating portion 214 is parallel to the second axis A2. For example, the second rotating portion 212, the third rotating portion 213, and the fourth rotating portion 214 may be arranged on a same side wall of the first mounting plate 123 and the first arm 13 to facilitate the installation of the first elastic mechanism 22.

The first elastic mechanism 22 may include a first elastic element 221 and a first cable 222. In some embodiments, the first elastic element 221 and the first cable 222 may be arranged as follows. Specifically, the first end of the first elastic element 221 is connected to the main turntable 121. A first end of the first cable 222 is fixed to the first rotating portion 211. A second end of the first cable 222 is wound around the second rotating portion 212, then wound around the fourth rotating portion 214 after being guided by the third rotating portion 213, and then connected to a second end of the first elastic element 221.

In other embodiments, the first elastic element 221 and the first cable 222 may also be arranged in such a way. Specifically, the first end of the first elastic element 221 is connected to the main turntable 121. The first end of the first cable 222 is fixed to the first rotating portion 211. The second end of the first cable 222 is wound around the fourth rotating portion 214, then wound around the second rotating portion 212 after being guided by the third rotating portion 213, and then connected to the second end of the first elastic element 221. Alternatively, the first end of the first elastic element 221 is connected to the main turntable 121. The first end of the first cable 222 is fixed to the first rotating portion 211. The second end of the first cable 222 is wound around the fourth rotating portion 214, then wound around the second rotating portion 212 after being guided by the third rotating portion 213, and then connected to the second end of the first elastic element 221.

Guiding the first cable 222 through the third rotating portion 213 may include that the first cable 222 is tangent to the third rotating portion 213, such that the direction change of the input device 10 about the second axis A2 is not affected. In the diagrammatic view illustrated in FIG. 8A, when the input device 10 is erect, that is, when the input device 10 as a whole does not rotate to the left or right relative to the base 12, the first cable 222 is tangent to the left and right sides of the third rotating portion 213. The above structural design allows the first elastic element 221 to provide a certain degree of compensation in the first degree of freedom for the corresponding gravity moment.

The first cable 222 in the present application may include a rigid cable. The rigid cable may include a cable that is not stretchable along the axial direction thereof. The first elastic element 221 may be an elastic element such as a spring.

Referring to FIG. 8B, which is a diagrammatic view of portions of the first gravity compensation mechanism in another embodiment.

In some embodiments, the first gravity compensation mechanism 20 may further include a first adjusting mechanism 23. The first adjusting mechanism 23 is connected between the second end of the first cable 222 and the second end of the first elastic element 221. The first adjusting mechanism 23 may include a first rotating member 231 and a second rotating member 232. The first rotating member 231 and the second rotating member 232 are rotatably connected to each other. For example, both of the first rotating member 231 and the second rotating member 232 are gear wheels that are engaged with each other, and a radius of the first rotating member 231 is smaller than that of the second rotating member 232. The second end of the first cable 222 is connected to the first rotating member 231, and the second end of the first elastic element 221 is connected to the second rotating member 232. Since the radius of the first rotating member 231 is smaller than that of the second rotating member 232, compared to the solution where the second end of the first cable 222 is directly connected to the second end of the first elastic element 221, the size of the first elastic element 221 may be smaller, thereby further achieving miniaturization of the input device to a certain extent. Of course, in other embodiments, the first rotating member 231 and the second rotating member 232 may also be other components other than gear wheels. For example, the first rotating member 231 and the second rotating member 232 may also be rotatably connected to each other by a conveyor belt or synchronous belt.

It should be noted that when the first arm 13 rotates to different positions, the force moment compensating for the parallelogram mechanism in the first degree of freedom is different. However, the gravity of each component of the parallelogram mechanism, the rotation angle of the first arm 13, and the rotation angle of the transmission turntable 181 are definite. Thus, the force moment in the first degree of freedom for compensating can be calculated when the first arm 13 is at any position.

The first elastic element 221 with a suitable elastic coefficient may be selected in present application as needed. For example, the larger the elastic coefficient of the elastic element, the greater the force moment that the gravity compensation mechanism can provide for compensating. Since the force moment for compensating for the parallelogram mechanism always changes during the rotation of the first arm 13, the force moment provided by the first gravity compensation mechanism 20 for compensating also changes when the first arm 13 rotates to different positions. However, the elastic coefficient of the first elastic element 221 and the distance between each two rotating portions are definite. Thus, when the first arm 13 is at any position, the force moment provided by the first gravity compensation mechanism 20 in the first degree of freedom, which is for compensating for the parallelogram mechanism, can be calculated.

In one embodiment, the first driving device 17 cooperates with the first gravity compensation mechanism 20 to compensate for the gravity moment of the parallelogram mechanism, and gravity moment is in the first degree of freedom that the parallelogram mechanism rotating about the second axis A2 as a whole. The surgical robot may further include a control device. The control device is used to couple with the first motor 171 and the first sensor of the first driving device 17 to receive, process, and send relevant commands. The control device controls the first motor 171 of the first driving device 17 to drive the first arm 13 to rotate about the second axis A2, and further controls the first driving device 17 to generate the force moment, which compensates for the gravity moment of the parallelogram mechanism in the first degree of freedom. The first degree of freedom is the parallelogram mechanism rotating about the second axis A2 as a whole.

Of course, in other embodiments, the gravity compensation may also be realized only by the first driving device 17 or the first gravity compensation mechanism 20.

The control device may be integrated into the input device 10. The control device may be integrated into the master console or slave operating device. The control device may also be independent from the master console and the slave operating device. The control device may be located locally or in the cloud. The control device may be composed of at least one controller, such as one, two, or multiple controllers.

It should be noted that the force moment provided by the first gravity compensation mechanism 20 is definite during the structural design and cannot be adjusted. However, the force moment provided by the first driving device 17 is adjustable as needed. Therefore, the cooperation of the first driving device 17 and the first gravity compensation mechanism 20 achieves precise compensation for the gravity moment of the parallelogram mechanism when the parallelogram mechanism rotates about the second axis A2, thereby reducing the operational burden of the operator.

Meanwhile, compared to the solution where only the first driving device 17 is used for gravity compensation, the present application reduces the compensation load of the first motor 171 of the first driving device 17 by the cooperation of the first driving device 17 and the first gravity compensation mechanism 20, which achieves miniaturization of the first motor 171 and facilitate the miniaturization of the input device 10. Compared to the solution where only the first gravity compensation mechanism 20 is used for gravity compensation, the present application improves the accuracy of gravity compensation by the cooperation of the first driving device 17 and the first gravity compensation mechanism 20.

In some embodiments, the compensation ratio of the first driving device 17 and the first gravity compensation mechanism 20 may be set as needed to match different application requirements. For example, the compensation ratio of the first driving device 17 and the first gravity compensation mechanism 20 may be 2:8, 3:7, and 4:6. Taking the compensation ratio of the first driving device 17 and the first gravity compensation mechanism 20 being 2:8 as an example, if the force moment to be compensated in the first degree of freedom is 100 Nm when the parallelogram mechanism is at a certain position, the first driving device 17 provides compensation of 20 Nm, and the first gravity compensation mechanism 20 provides compensation of 80 Nm. The compensation ratio of the first gravity compensation mechanism 20 may be adjusted by selecting the first elastic element 221 with different elastic coefficients. The higher the elastic coefficient of the first elastic element 221, the higher the compensation ratio of the first gravity compensation mechanism 20.

In one embodiment, the compensation ratio of the first gravity compensation mechanism 20 and the first driving device 17 is 8:2. That is, the first gravity compensation mechanism 20 provides compensation for a large amount of gravity, and the first driving device 17 provides compensation for a small amount of gravity. As such, accurate compensation for gravity is ensured, and the load of the first motor 171 of the first driving device 17 is reduced. Thus, the size of the first motor 171 may be reduced to achieve miniaturization of the input device 10, thereby reducing the operational burden of the operator.

In one embodiment, when the first arm 13 is at a certain position, the force moment that needs to be compensated by the first driving device 17 is equal to the difference between the force moment that needs to be compensated for and the force moment compensated by the first gravity compensation mechanism 20. Thus, no matter the first arm 13 rotates to any position, the first driving device 17 and the first gravity compensation mechanism 20 cooperate with each other to generate the force moment. The force moment balance the gravity moment of the parallelogram mechanism in the first degree of freedom, and the first degree of freedom is the parallelogram mechanism rotating about the second axis A2 as a whole.

For example, the first sensor is coupled to the control device. The control device obtains the position change of the first arm 13 through the first sensor of the first driving device 17, thereby obtaining the force moment that needs to be compensated for when the first arm 13 is at the corresponding position. The control device controls the second motor 191 to cooperate with the gravity compensation mechanism to generate the force moment, and the force moment balances the gravity moment of the parallelogram mechanism.

In some embodiments, the first gravity compensation mechanism 20 may further include a first force sensor. The first force sensor is coupled to the control device. The first force sensor senses an actual force moment compensated by the first gravity compensation mechanism 20. Then, the control device determines the force moment that needs to be compensated by the first driving device 17, which is equal to the different between the force moment that needs to be compensated when the first arm 13 is at the corresponding position and the actual force moment compensated by the first gravity compensation mechanism 20 at that position.

It should be noted that after a long period of use, some components of the first gravity compensation mechanism 20 may experience aging. For example, the elastic coefficient of the first elastic element 221 may change. As a result, the force moment compensating for the parallelogram mechanism by the first gravity compensation mechanism 20 will decrease. However, if the force moment that needs to be compensated by the first driving device 17 is still determined by calculation, the calculated force moment of the first driving device 17 will be smaller than the force moment actually needed, resulting in insufficient compensation for the parallelogram.

The embodiment monitors the actual force moment provided by the first gravity compensation mechanism 20 in real time, and determines the force moment that needs to be compensated by the first driving device 17 based on the actual force moment provided by the first gravity compensation mechanism 20, thereby avoiding an inaccurate calculation of the force moment of the first gravity compensation mechanism 20 caused by the aging of the first gravity compensation mechanism 20. Thus, accurate compensation for the gravity moment of the parallelogram mechanism is ensured, thereby improving the user experience.

Of course, in some embodiments, the control device may also compare the actual force moment compensated by the first gravity compensation mechanism 20 sensed by the first sensor with the calculated force moment of the first gravity compensation mechanism 20. If the difference between the two values exceeds a predetermined value, the user is alerted that the first gravity compensation mechanism 20 has failed or needs to be replaced.

Referring to FIG. 11, the input device 10 may further include a second gravity compensation mechanism 30. The second gravity compensation mechanism 30 is connected between the base 12 and the transmission turntable 181, and generates a force moment in a second degree of freedom, wherein the second degree of freedom is the rotation about the third axis A3, and the force moment balances the gravity moment of the parallelogram mechanism. In some embodiments, the second gravity compensation mechanism 30 may include a second rotating mechanism 31 and a second elastic mechanism 32. The second rotating mechanism 31 may include multiple rotating portions. The rotating portions are distributed on the second mounting plate 124 and the transmission turntable 181 of the base 12.

For ease of understanding, the second rotating mechanism 31 may include a first part of rotating portions and a second part of rotating portions. For example, the first part of rotating portions is located on the second mounting plate 124, and the second part of rotating portion is located on the transmission turntable 181. The second elastic mechanism 32 is coupled between the main turntable 121 of the base 12 and the second rotating mechanism 31, and generates the force moment in the second degree of freedom that balances the gravity moment of the parallelogram mechanism. Thus, the operator is allowed to easily pull the input device 10 in the second degree of freedom.

The second rotating mechanism 31 may include a fifth rotating portion 311, a sixth rotating portion 312, and a seventh rotating portion 313. For example, the fifth rotating portion 311 may include a fifth pulley, the sixth rotating portion 312 may include a sixth pulley, and the seventh rotating portion 313 may include a seventh pulley. The fifth rotating portion 311 is fixed to the second mounting plate 124 through a connecting member. Each of the sixth rotating portion 312 and the seventh rotating portion 313 is rotatably connected to the transmission turntable 181.

The rotation axis of the sixth rotating portion 312 coincides with the second axis A2. The rotation of the sixth rotating portion 312 is independent from the rotation of the first arm 13 about the second axis A2. The rotation axis of the seventh rotating portion 313 is parallel to the second axis A2. The sixth rotating portion 312 and the seventh rotating portion 313 may also be arranged on a same side of the second mounting plate 124 and the transmission turntable 181.

When the input device 10 includes the first gravity compensation mechanism 20 and the second gravity compensation mechanism 30, the rotating portions of the first rotating mechanism 21 and the rotating portions of the second rotating mechanism 31 are arranged on different sides of the input device 10, thereby avoiding an inaccurate compensation of the force moment caused by mutual interference between the elastic elements and the cables.

The second elastic mechanism 32 may include a second elastic element 321 and a second cable 322. In some embodiments, the second elastic element 321 and the second cable 322 may be set as follows. Specifically, a first end of the second elastic element 321 is connected to the main turntable 121. A first end of the second cable 322 is fixed to the fifth rotating portion 311. A second end of the first cable 222 is wound around the seventh rotating portion 313, and then connected to a second end of the second elastic element 321 after being guided by the sixth rotating portion 312. Alternatively, the first end of the second cable 322 is fixed to the fifth rotating portion 311. The second end of the first cable 222 is wound around the seventh rotating portion 313, and then connected to the second end of the second elastic element 321 after being guided by the sixth rotating portion 312.

Of course, in other embodiments, the second rotating mechanism 31 may further include an eighth rotating portion. The eighth rotating portion is coaxial with and rotates relative to the fifth rotating portion 311. The second end of the second cable 322 is wound around the eighth rotating portion and finally connected to the second end of the second elastic element 321.

Guiding the second cable 322 by the sixth rotating portion 312 may include that the second cable 322 is tangent to the sixth rotating portion 312 and wound around the sixth rotating portion 312. The above structural design uses the second elastic element 321 to provide a certain degree of compensation in the third degree of freedom for the gravity moment.

In the embodiment, the input device 10 include both of the first gravity compensation mechanism 20 and the second gravity compensation mechanism 30. Thus, the two gravity compensation mechanisms provide compensation in two degrees of freedom for the gravity moments of the parallelogram mechanism, thereby allowing the user to easily pull the input device 10 in the two degrees of freedom. Of course, in other embodiments, the input device 10 may only include the first gravity compensation mechanism 20 or the second gravity compensation mechanism 30. Alternatively, the input device 10 may not include any gravity compensation mechanism.

The second cable 322 in the present application may include a rigid cable. The rigid cable may include a cable that is not stretchable cable along the axial direction thereof. The second elastic element 321 may be an elastic element such as a spring.

In some embodiments, the second gravity compensation mechanism may further include a second adjusting mechanism. The structure of the second adjusting mechanism is substantially the same as that of the first adjusting mechanism, which will not be repeated. The second adjusting mechanism is connected between the second end of the second cable and the second end of the second elastic element. Compared to the solution where the second end of the second cable is directly connected to the second end of the second elastic element, the size of the second elastic element may be smaller, which further achieves miniaturization of the input device to a certain extent.

It should be noted that when the transmission turntable 181 rotates to different positions, the force moment compensating for the parallelogram mechanism in the second degree of freedom is different. However, the gravity of each component of the parallelogram mechanism, the rotation angle of the first arm 13, and the rotation angle of the transmission turntable 181 are definite. Thus, the force moment in the second degree of freedom for compensating may be calculated when the transmission turntable 181 is at different positions.

The second elastic element 321 with a suitable elastic coefficient may be selected in present application as needed. The elastic coefficient of the second elastic element 321 and the distance between each two rotating portions are definite. Thus, when the transmission turntable 181 is at any position, the force moment provided by the second gravity compensation mechanism 30 in the second degree of freedom, which is for compensating for the parallelogram mechanism, can be calculated.

In one embodiment, the second driving device 19 cooperates with the second gravity compensation mechanism 30 to compensate for the gravity moment of the parallelogram mechanism, and gravity moment is in the second degree of freedom. The control device is further coupled with the second motor 191 and the second sensor of the second driving device 19 to receive, process, and send relevant commands. The control device controls the second motor 191 of the second driving device 19 to drive the transmission turntable 181 to rotate about the second axis A2, thereby driving the second arm 14 to rotate about the third axis A3. At the same time, the control device controls the second driving device 19 to generate the force moment, which compensates for the gravity moment of the parallelogram mechanism in the second degree of freedom. The second degree of freedom is the parallelogram mechanism rotating about the third axis A3 as a whole.

The manner and effect of the second driving device 19 and the second gravity compensation mechanism 30 cooperating to compensate for the gravity moment are substantially the same as those of the first driving device 17 and the first gravity compensation mechanism 20 cooperating to compensate for the gravity moment, which will not be repeated.

In some embodiments, the second gravity compensation mechanism 30 may further include a second force sensor. The second force sensor is coupled to the control device. The second force sensor senses an actual force moment compensated by the second gravity compensation mechanism 30. Then, the control device determines that the force moment that needs to be compensated by the second driving device 19, which is equal to the different between the force moment that needs to be compensated when the first arm 13 is at the corresponding position and the actual force moment compensated by the second gravity compensation mechanism 30 at that position.

It should be noted that after a long period of use, some components of the second gravity compensation mechanism 30 may experience aging. For example, the elastic coefficient of the first elastic element 221 may change. As a result, the force moment compensated for the parallelogram mechanism by the second gravity compensation mechanism 30 will decrease. However, if the force moment that needs to be compensated by the second driving device 19 is still determined by calculation, the calculated force moment of the second driving device 19 will be smaller than the force moment actually needed, resulting in insufficient compensation for the parallelogram.

The embodiment monitors the actual force moment provided by the second gravity compensation mechanism 30 in real time, and determines the force moment that needs to be compensated by the second driving device 19 based on the actual force moment provided by the second gravity compensation mechanism 30. Thus, accurate compensation for the gravity moment of the parallelogram mechanism is ensured, thereby improving the user experience..

Of course, in some embodiments, the control device may also compare the actual force moment compensated by the second gravity compensation mechanism 30 sensed by the second sensor with the calculated force moment of the second gravity compensation mechanism 30. If the difference between the two values exceeds a predetermined value, the user is alerted that the second gravity compensation mechanism 30 has failed or needs to be replaced.

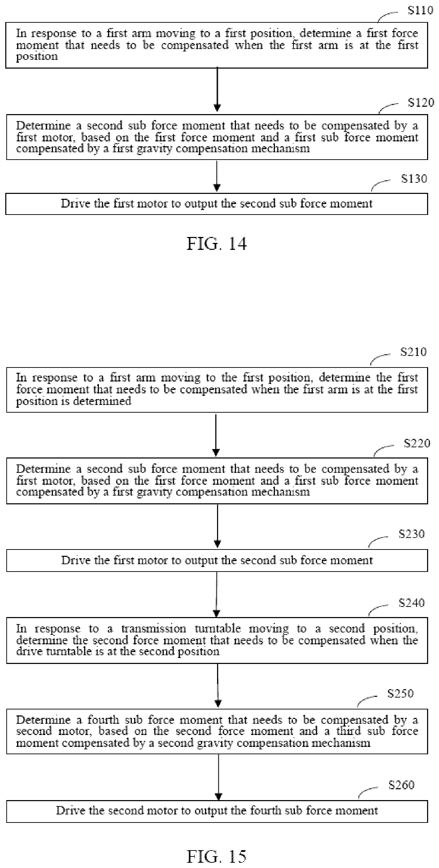

Referring to FIG. 14, which is a flowchart of a control method for the input device 10. The method is executed by the control device to control the input device 10 mentioned above.

The control method for the input device 10 may include the following steps.

S110: In response to the first arm 13 moving to a first position, a first force moment that needs to be compensated when the first arm 13 is at the first position is determined.

In some embodiments, also referring to FIG. 4, the first arm 13 is equipped with a first sensor. The first sensor senses any position change of the first arm 13 when the first arm 13 rotates about the second axis A2. When the first arm 13 moves to the first position, the first sensor sends information as to the first position of the first arm 13 to the control device, and alternatively, the control device obtains the first position information of the first arm 13 from the first sensor. The control device, in response to the information as to the first position, determines a first force moment that needs to be compensated when the first arm 13 is at the first position. Of course, in other embodiments, the position change of the first arm 13 may also be input by other input devices. The control device may obtain the information as to the first position input by the other input devices and determine the first force moment.

It should be noted that the first position may be any position reached after the first arm has moved.

In some embodiments, the control device determines the first force moment $M_{AT}$ that needs to be compensated when the first arm 13 is at the first position. The first force moment $M_{AT}$ is obtained according to the following formula:

$$M_{AT}=(G_1*L1+G_3*L3+G_4*L4)*\sin\theta_1+M_{x1}$$

Wherein, $G_1$ is a gravity of the transmission turntable 181, $G_3$ is a gravity of the transmission arm 182, $G_4$ is a gravity of the second arm 14, $\theta_1$ may be the rotating angle of the parallelogram mechanism as a whole rotating relative to the base 12, i.e., $\theta_1$ is associated with the first degree of freedom. The first degree of freedom is independent from the second degree of freedom. L1 is a distance from a center of gravity of the first arm 13 to the end of the first arm 13 connected to the first mounting plate 123, L3 is a distance from the end of the first arm 13 connected to the second arm 14 to a center of gravity of the transmission arm 182, and L4 is a distance from the end of the transmission arm 182 connected to the transmission turntable 181 to the end of the transmission turntable 181 connected to the second mounting plate 124. $M_{x1}$ is the gravity moment of the operating assembly 15 located on the second arm 14 in the first degree of freedom. Since the operating assembly 15 may be at different positions, the corresponding value of $M_{x1}$ is also different, but the specific value of $M_{x1}$ may be calculated.

Since $(G_1*L1+G_3*L3+G_4*L4)$ and $M_{x1}$ are known, the gravity moment of the parallelogram mechanism in the first degree of freedom is a sine function with respect to the variable $\theta_1$. The variable $\theta_1$ is associated with the change in the gravity moment of the parallelogram mechanism in the first degree of freedom (i.e., the degree of freedom of the parallelogram mechanism rotating relative to the base 12 as a whole). The information as to the first position of the first arm 13, that is, the value $\theta_1$, is obtained from the first sensor. By substituting the value $\theta_1$ sensed by the first sensor into the above formula, the first force moment that needs to be compensated when the first arm 13 is at the first position is calculated. Of course, the first force moment $M_{AT}$ that needs to be compensated when the first arm 13 is at any position may be calculated and stored in advance. When the value $\theta_1$ sensed by the first sensor is obtained, the first force moment corresponding to the value $\theta_1$ may be determined by the stored data.

Of course, in the case where the input device 10 only includes the base 12, the first arm 13, the first motor 171, the first sensor, and the first gravity compensation mechanism 20, the first position of the first arm 13 may also be obtained by the first sensor. Based on the information as to the first position, the first force moment that needs to be compensated when the first arm 13 is in the first position may be calculated.

S120: a second sub force moment that needs to be compensated by the first motor 171 is determined, based on the first force moment and a first sub force moment compensated by the first gravity compensation mechanism 20.

In some embodiments, also referring to FIG. 8A, before determining the second sub force moment that needs to be compensated by the first motor 171 based on the first force moment and the first sub force moment compensated by the first gravity compensation mechanism 20, the control method may further include obtaining the first sub force moment compensated by the first gravity compensation mechanism 20 at the first position. For example, the control device, in response to the information as to the first position, determines the first sub force moment compensated by the first gravity compensation mechanism 20.

In some embodiments, the control device determines the first sub force moment $M_{A1}$ compensated by the first gravity compensation mechanism 20 at the first position, according to the following formula:

$$M_{A1}=k1*a1*b1*\sin\theta_1$$

Wherein, k1 is the elastic coefficient of the first elastic element 221, a1 is a distance from the rotation axis of the fourth rotating portion 214 to the rotation axis of the third rotating portion 213, and b1 is a distance from the rotation axis of the third rotating portion 213 to the rotation axis of the second rotating portion 212.

Since k1*a1*b1 is known, the first sub force moment compensated by the first gravity compensation mechanism 20 is a sine function with respect to the variable $\theta_1$. The information as to the first position of the first arm 13, that is, the value $\theta_1$, is obtained from the first sensor. By substituting the value $\theta_1$ sensed by the first sensor into the above formula, the first sub force moment compensated by the first gravity compensation mechanism 20 at the first position is calculated. Of course, the first sub force moment compensated by the first gravity compensation mechanism 20 when the first arm 13 is at any position may be calculated and stored in advance. When the value $\theta_1$ sensed by the first sensor is obtained, the first sub force moment corresponding to the value $\theta_1$ may be determined by the stored data.

In one embodiment, by substituting the first force moment and the first sub force moment compensated by the first gravity compensation mechanism 20 into the following formula, the second sub force moment $M_{A2}$ that needs to be compensated by the first motor 171 is calculated. The formula is as follows:

$$M_{A2}=M_{AT}-M_{A1}$$

Of course, in other embodiments, the second sub force moment that needs to be compensated by the first motor 171 may also be determined based on the compensation ratio between the first gravity compensation mechanism 20 and the first motor 171. For example, if the compensation ratio between the first gravity compensation mechanism 20 and the first motor 171 is 8:2, the second sub force moment that needs to be compensated by the first motor 171 is 1/5 $M_{AT}$. It should be noted that the compensation ratio between the first gravity compensation mechanism 20 and the first motor 171 may be set during the structural design of the input device 10. For example, the first sub force moment that needs to be compensated by the first gravity compensation mechanism 20 is set to be 80% of the first force moment. When designing the components of the first gravity compensation mechanism 20, the parameters of each component or between two components should allow the value k1*a1*b1*sin$\theta_1$ to be equal to 0.8*$M_{AT}$.

In some embodiments, since the first arm and the first motor are rotatably connected to each other, when the parallelogram mechanism is pulled by the operator in the first degree of freedom, the first motor will also rotate accordingly. During the rotation of the first motor, the first motor will output different currents under different forces. The control device may the output current of the first motor and calculate the force moment that needs to be compensated by the first motor accordingly. For example, the parallelogram mechanism may be arbitrarily pulled, such that the current change output by the first motor is obtained when the first arm is located at different positions. The force moment that needs to be compensated by the first motor at different positions is obtained based on the current change. Then, when the first arm moves to the corresponding position, the first motor is driven to provide the corresponding force moment for compensating.

In other embodiments, the first gravity compensation mechanism 20 may further include a first force sensor. The first force sensor is coupled to the control device. The first force sensor senses the first sub force moment actually compensated by the first gravity compensation mechanism 20. The control device determines the second sub force moment that needs to be compensated by the first driving device 17, which is the difference between the first force moment that needs to be compensated when the first arm 13 is at that position and the first sub force moment actually compensated by the first gravity compensation mechanism 20 at that position.

One embodiment monitors the first sub force moment actually compensated by the first gravity compensation mechanism 20 in real time, and calculates the second sub force moment that needs to be compensated by the first driving device 17 according to the first sub force moment actually compensated by the first gravity compensation mechanism 20, thereby avoiding inaccurate calculation of the first sub force moment caused by the aging of the first gravity compensation mechanism 20, ensuring accurate compensation for the gravity moment of the parallelogram mechanism in the first degree of freedom, and improving the user experience.

Of course, the first motor may also periodically identify whether the force moment compensated by the first gravity compensation mechanism at different positions changes, and regularly correct the first sub force moment of the first gravity compensation mechanism, thereby ensuring accurate compensation for the gravity moment of the quadrilateral mechanism in the first degree of freedom, and improving the user experience.

S130: the first motor 171 is driven to output the second sub force moment.

In some embodiments, after the control device obtains the second sub force moment that the first motor 171 needs to output, the control device controls the first motor 171 to output the second sub force moment. Thus, the first motor 171 cooperates with the first gravity compensation mechanism 20 to compensate for the gravity during the rotation of the first arm 13 about the second axis A2 (the first degree of freedom).

Since the first sub force moment compensated by the first gravity compensation mechanism 20 is definite during structural design, the first sub force moment cannot be flexibly adjusted during use. However, the second sub force moment compensated by the first driving device 17 is adjustable as needed. Therefore, the cooperation of the first driving device 17 and the first gravity compensation mechanism 20 achieves precise compensation for the gravity of the parallelogram mechanism in the first degree of freedom, thereby reducing the operational burden of the user.

In some embodiments, the control method for the input device 10 may further include the control device comparing the first sub force moment actually compensated by the first gravity compensation mechanism 20 sensed by the first sensor with the first sub force moment that compensated by the first gravity compensation mechanism 20 obtained by calculation. If the difference between the two values exceeds a predetermined value, the user is alerted that the first gravity compensation mechanism 20 has failed or needs to be replaced.

Referring to FIG. 15, which is another flowchart of a control method for the input device 10. The control method is executed by the control device to control the input device 10 mentioned above.

The control method for the input device 10 may include the following steps:

S210: In response to the first arm 13 moving to the first position, the first force moment that needs to be compensated when the first arm 13 is at the first position is determined.

S220: the second sub force moment that needs to be compensated by the first motor 171 is determined, based on the first force moment and the first sub force moment compensated by the first gravity compensation mechanism 20.

S230: the first motor 171 is driven to output the second sub force moment.

It should be noted that the specific content of step S210 is substantially the same as that of step S110, which will not be repeated. The specific content of steps S220 is substantially the same as that of S120, which will not be repeated. The specific content of steps S230 is substantially the same as that of S130, which will not be repeated.

S240: In response to the transmission turntable 181 moving to the second position, the second force moment that needs to be compensated when the drive turntable 181 is at the second position is determined.

In some embodiments, also referring to FIG. 4, the transmission turntable 181 is provided with that senses any position change of the transmission turntable 181 rotating about the second axis A2. When the transmission turntable 181 moves to the second position, the second sensor sends an information as to the second position of the transmission turntable 181 to the control device, and alternatively, the control device obtains the information as to the second position of the transmission turntable 181 from the second sensor. The control device, in response to the information as to the second position, determines the first force moment that needs to be compensated when the transmission turntable 181 is at the second position. Of course, in other embodiments, the position change of the transmission turntable 181 may also be input by other input devices. The control device may determine the second force moment according to the information as to the second position input by the other input devices.

It should be noted that the second position is any position reached after the transmission turntable has moved.

In some embodiments, the control device determines the second force moment $M_{BT}$ that needs to be compensated when the transmission turntable 181 is at the second position, based on the following formula:

$$M_{BT}=(G_2* L2'+ G_3* L3'+ G_4* L4')*\sin\theta_2 +M_{x2}$$

Wherein, $G_2$ is the gravity of the first arm 13, $G_3$ is the gravity of the transmission arm 182, and $G_4$ is the gravity of the second arm 14. $\theta_2$ may be the angle between adjacent linkage rods of the parallelogram mechanism after the rotation, i.e., $\theta_2$ is associated with the second degree of freedom. The first degree of freedom is independent from the second degree of freedom. L2' is a distance between the center of gravity of the transmission turntable 181 and the end of the transmission turntable 181 connected to the second mounting plate 124, L3' is a distance between the end of the first arm 13 connected to the second arm 14 and the end of the first arm 13 connected to the first mounting plate 123, and L4' is the distance between the end of the transmission arm 182 connected to the transmission turntable 181 and the center of gravity of the second arm 14. $M_{x2}$ is the gravity moment of the operating assembly 15 located on the second arm 14 in the second degree of freedom. Since the operating assembly 15 may be at different positions, the corresponding value of $M_{x2}$ is also different, but the specific value of $M_{x2}$ may be calculated.

Since $(G_2*L2'+G_3*L3'+G_4*L4')$ and $M_{x2}$ are known, the gravity moment of the parallelogram mechanism in the second degree of freedom is a sine function with respect to the variable $\theta_2$. The variable $\theta_2$ is associated with the change in the gravity moment of the parallelogram mechanism in the second degree of freedom (i.e., the degree of freedom of relative rotation of the internal linkage rods of the parallelogram mechanism). The information as to the second position of the transmission turntable 181, that is, the value $\theta_2$, is obtained through the second sensor. By substituting the value $\theta_2$ sensed by the second sensor into the above formula, the second force moment that needs to be compensated when the transmission turntable 181 is at the second position is calculated. Of course, the second force moment that needs to be compensated when the transmission turntable 181 is at any position may be calculated and stored in advance. When the value $\theta_2$ sensed by the second sensor is obtained, the second force moment corresponding to the value $\theta_2$ may be determined by the stored data.

S250: the fourth sub force moment that needs to be compensated by the second motor 191 is determined, based on the second force moment and the third sub force moment compensated by the second gravity compensation mechanism 30.

In some embodiments, also referring to FIG. 11, before determining the fourth sub force moment based on the second force moment and the third sub force moment, the control method may further include obtaining the third sub force moment compensated by the second gravity compensation mechanism 30 at the second position. For example, the control device, in response to the information as to the second position, determines the third sub force moment compensated by the second gravity compensation mechanism 30 at the second position.

In some embodiments, the control device determines the third sub force moment $M_{B1}$ compensated by the second gravity compensation mechanism 30 at the second position, according to the following formula:

$$M_{B1}=k2*a2*b2*\sin\theta_2$$

Wherein, k2 is the elastic coefficient of the second elastic element 321, a2 is a distance from the rotation axis of the seventh rotating portion 313 to the second axis A2, and b2 is a distance from the rotation axis of the sixth rotating portion 312 to the rotation axis of the fifth rotating portion 311.

Since k2*a2*b2 is known, the third sub force moment of the second gravity compensation mechanism 30 is a sine function with respect to the variable $\theta_2$. The information as to the second position of the transmission turntable 181, that is, the value $\theta_2$, is obtained through the second sensor. By substituting the value $\theta_2$ sensed by the second sensor into the above formula, the third sub force moment compensated by the second gravity compensation mechanism 30 at the second position is calculated. Of course, the third sub force moment compensated by the second gravity compensation mechanism 30 at any position of the transmission turntable 181 may also be calculated and stored in advance. When the value $\theta_2$ sensed by the second sensor is obtained, the third sub force moment corresponding to the value $\theta_2$ may be determined by the stored data.

In one embodiment, by substituting the second force moment and the third sub force moment into the following formula, the fourth sub force moment $M_{B2}$ that needs to be compensated by the second motor 191 may be calculated. The formula is as follows:

$$M_{B2}=M_{BT}-M_{B1}$$

Of course, in other embodiments, the fourth sub force moment that needs to be compensated by the second motor 191 may also be determined based on the compensation ratio between the second gravity compensation mechanism 30 and the second motor 191. For example, if the compensation ratio between the second gravity compensation mechanism 30 and the second motor 191 is 8:2, the fourth sub force moment that needs to be compensated by the second motor 191 is 1/5 $M_{BT}$. It should be noted that the compensation ratio between the second gravity compensation mechanism 30 and the second motor 191 may be set during the structural design of the input device 10. For example, the third sub force moment that needs to be compensated by the second gravity compensation mechanism 30 is set to be 80% of the first force moment. When designing the components of the second gravity compensation mechanism 30, the parameters of each component or between two components should allow the value k2*a2*b2* $\sin\theta_2$ to be equal to 0.8*$M_{BT}$.

In some embodiments, since the transmission turntable and the second motor are the rotatably connected to each other, when the parallelogram mechanism is pulled by the operator in the second degree of freedom, the second motor will also rotate accordingly. During the rotation of the second motor, the second motor will output different currents under different forces. The control device may obtain the output current of the second motor and calculate the force moment that needs to be compensated by the second motor accordingly. For example, the parallelogram mechanism may be arbitrarily pulled, such that the current change output by the second motor is obtained when the transmission turntable is at different positions. The force moment that needs to be compensated by the second motor at different positions is obtained based on the current change. Then, when the transmission turntable moves to the corresponding position, the second motor is driven to provide the corresponding force moment for compensating.

In other embodiments, the second gravity compensation mechanism 30 may further include a second force sensor. The second force sensor is coupled to the control device. The second force sensor senses the third sub force moment actually compensated by the second gravity compensation mechanism 30. The control device determines the fourth sub force moment that needs to be compensated by the second driving device 19, which is equal to the difference between the second force moment that needs to be compensated when the second arm 14 is at that position and the third sub force moment actually compensated by the second gravity compensation mechanism 30 at that position. One embodiment monitors the third sub force moment actually compensated by the second gravity compensation mechanism 30 in real time, and calculates the fourth sub force moment that needs to be compensated by the second driving device 19 according to the third sub force moment actually compensated by the second gravity compensation mechanism 30, thereby avoiding inaccurate calculation of the third sub force moment caused by the aging of the second gravity compensation mechanism 30, ensuring accurate compensation for the gravity moment of the parallelogram mechanism in the second degree of freedom, and improving the user experience.

Of course, the second motor may also periodically identify whether the force moment compensated by the second gravity compensation mechanism at different positions changes, and regularly correct the third sub force moment of the second gravity compensation mechanism, thereby ensuring accurate compensation for the gravity moment of the quadrilateral mechanism in the second degree of freedom, and improving the user experience.

S260: the second motor 191 is driven to output the fourth sub force moment.

In some embodiments, after the control device obtains the fourth sub force moment that the second motor 191 needs to output, the control device controls the second motor 191 to output the fourth sub force moment. Thus, the second motor 191 cooperates with the second gravity compensation mechanism 30 to compensate for the gravity of the transmission shaft in the second degree of freedom during the rotation about the second axis A2.

Since the third sub force moment compensated by the second gravity compensation mechanism 30 is definite during structural design, the third sub force moment cannot be flexibly adjusted during use. However, the fourth sub force moment compensated by the second driving device 19 is adjustable as needed. Therefore, the cooperation of the second driving device 19 and the second gravity compensation mechanism 30 achieves precise compensation for the gravity of the parallelogram mechanism in the second degree of freedom, thereby reducing the operational burden of the user.

The control method of the present application compensates for the gravity of the parallelogram mechanism in the first degree of freedom through the cooperation of the first driving device 17 and the first gravity compensation mechanism 20, and compensates for the gravity of the parallelogram mechanism in the second degree of freedom through the cooperation of the second driving device 19 and the second gravity compensation mechanism 30, thereby ensuring the miniaturization of the input device 10, improving the flexibility of the operation of the input device 10, and enabling the user to easily pull the input device 10 in both two degrees of freedom.

In some embodiments, the control method for the input device 10 may further include the control device comparing the third sub force moment actually compensated by the second gravity compensation mechanism 30 sensed by the second sensor with the third sub force moment that compensated by the second gravity compensation mechanism 30 obtained by calculation. If the difference between the two values exceeds a predetermined value, the user is alerted that the second gravity compensation mechanism 30 has failed or needs to be replaced.

The above embodiments are only for describing but not intended to limit the present disclosure. Although the embodiments of the present disclosure have been described, those having ordinary skill in the art can understand that changes may be made within the principles of the present disclosure, up to and including the full extent established by the broad general meaning of the terms used in the claims. It will, therefore, be appreciated that the embodiments described above may be modified within the scope of the claims.

## Claims

1. An input device, **characterized by** comprising a base, a first arm, a second arm, a first motor, a first transmission mechanism, and an operating assembly;

   wherein the base, the first arm, the second arm, and the operating assembly are sequentially and rotatably connected to each other, the operating assembly is configured to receive operations of an operator, the first arm and the second arm are configured to rotate to allow the operating assembly to move in at least two degrees of freedom;
   the first motor is mounted on the base, the first arm comprises an arm turntable, the arm turntable is rotatably connected to an output shaft of the first motor through the first transmission mechanism, the output shaft is configured to rotate to drive the arm turntable to rotate, such that the first arm rotates relative to the base, and a rotation speed of the output shaft is greater than a rotation speed of the arm turntable.

2. The input device according to claim 1, **characterized in that**, the first transmission mechanism comprises a first transmission member, the first transmission member is connected between the arm turntable and the output shaft of the first motor, and radius of the arm turntable is greater than a radius of the output shaft of the first motor.

3. The input device according to claim 1, **characterized in that**, the first transmission mechanism comprises a first transmission member, a first adjusting member, and a second transmission member, the first transmission member is rotatably connected between the output shaft and an input portion of the first adjusting member, the second transmission member is connected between an output portion of the first adjusting member and the arm turntable, and a radius of the input portion is larger than a radius of the output portion.

4. The input device according to any one of claims 1-3, **characterized in that**, the input device further comprises a second motor and a second transmission mechanism, the second motor is mounted on the base, and an output shaft of the second motor is rotatably connected to the second arm through the second transmission mechanism.

5. The input device according to claim 4, **characterized in that**, the input device further comprises a transmission turntable and a transmission arm, the transmission turntable is rotatably connected to the base, the transmission arm is rotatably connected between the transmission turntable and the second arm, the second motor is configured to drive the transmission turntable to rotate through the second transmission mechanism, the transmission turntable is configured to drive the transmission arm to move when the transmission turntable rotates, such that the second arm rotates relative to the first arm.

6. The input device according to claim 5, **characterized in that**, the base comprises a main body, a first mounting plate, and a second mounting plate, the first mounting plate and the second mounting plate are connected to the main body and opposite to each other, the arm turntable and the first motor are mounted on the first mounting plate and spaced from each other, and the transmission turntable and the second motor are mounted on the second mounting plate and spaced from each other.

7. The input device according to claim 6, **characterized in that**, the second motor is parallel to the first motor.

8. The input device according to claim 6 or 7, **characterized in that**, one end of the first motor near the second mounting plate extends through the second mounting plate.

9. The input device according to claim 7, **characterized in that**, one end of the second motor near the first mounting plate extends through the first mounting plate.

10. The input device according to claim 6, **characterized in that**, the input device further comprises a fixing shaft, the fixing shaft extends through and is fixed to each of the first mounting plate and the second mounting plate, and two ends of the fixing shaft are rotatably connected to the arm turntable and the transmission turntable, respectively.

11. The input device according to claim 6, **characterized in that**, the input device further comprises a connecting member, a main motor, and a main transmission mechanism, the base further comprises a main turntable, the main turntable is connected to the main body, the main motor is mounted on the connecting member, and an output shaft of the main motor is rotatably connected to the main turntable through the main transmission mechanism.

12. The input device according to claim 11, **characterized in that**, an extension line of the main motor in a length direction thereof is perpendicular to a rotation axis of the arm turntable, and an extension line of each of the first motor and the second motor in a length direction thereof is parallel to the rotation axis of the arm turntable.

13. The input device according to claim 1, **characterized in that**, the input device further comprises a first gravity compensation mechanism connected between the base and the first arm, and the first gravity compensation mechanism is configured to generate a force moment to balance a gravity moment of relevant components of the input device.

14. The input device according to claim 13, **characterized in that**, the first gravity compensation mechanism comprises a plurality of rotating portions, a first cable, and a first elastic element, at least a portion of the rotating portions is fixed to the arm turntable, an end of the first elastic element is fixed to the base, another end of the first elastic element is fixed to the first cable, an end of the first cable away from the first elastic element is engaged with the rotating portions and fixed to one of the rotating portions.

15. The input device according to claim 13, **characterized in that**, the input device further comprises a first sensor, the first sensor is configured to sense position change of the first arm, and the first motor is configured to cooperate with the first gravity compensation mechanism to generate the force moment to balance the gravity moment of the relevant components of the input device.

16. An input device, **characterized by** comprising:

   a base;
   a first arm rotatably connected to the base;
   a first motor, an output shaft of the first motor is configured to rotate to drive the first arm to rotate relative to the base;
   a first gravity compensation mechanism connected between the base and the first arm; and
   a control device coupled with the first motor, wherein the control device is configured to:

      responding to the first arm moving to a first position, and determine a first moment that needs to be compensated when the first arm is at the first position;
      determine a second sub force moment that needs to be compensated by the first motor, based on the first force moment and a first sub force moment compensated by the first gravity compensation mechanism; and
      drive the first motor to output the second sub force moment.

17. The input device according to claim 16, **characterized in that**, the input device further comprises a first sensor, the first sensor is coupled with the control device and configured to sense position change of the first arm;
wherein responding to the first arm moving to the first position comprises:
responding to an information as to the first position sensed by the first sensor.

18. The input device according to claim 16, **characterized in that**, the input device further comprises a first force sensor, the first force sensor is coupled with the control device and configured to sense a first sub force moment actually compensated by the first gravity compensation mechanism, and determining the second sub force moment that needs to be compensated by the first motor comprising:

obtaining the first sub force moment through the first force sensor; and
determining the second sub force moment through following formula, in which the second sub force moment is equal to the difference between the first force moment and the first sub force moment.

19. The input device according to claim 16, **characterized in that**, the input device further comprises:
determining the second sub force moment based on a compensation ratio of the first motor and the first gravity compensation mechanism.

20. A surgical robot, **characterized by** comprising the input device according to any one of claims 1-15 or the input device according to any one of claims 16-19.

200

FIG. 1

FIG. 2

10

FIG. 3

FIG. 4

FIG. 5

FIG. 6

FIG. 7

FIG. 8A

FIG. 8B

FIG. 9

FIG. 10A

FIG. 10B

FIG. 11

FIG. 12

FIG. 13

S110

In response to a first arm moving to a first position, determine a first force moment that needs to be compensated when the first arm is at the first position

S120

Determine a second sub force moment that needs to be compensated by a first motor, based on the first force moment and a first sub force moment compensated by a first gravity compensation mechanism

S130

Drive the first motor to output the second sub force moment

FIG. 14

S210

In response to a first arm moving to the first position, determine the first force moment that needs to be compensated when the first arm is at the first position is determined

S220

Determine a second sub force moment that needs to be compensated by a first motor, based on the first force moment and a first sub force moment compensated by a first gravity compensation mechanism

S230

Drive the first motor to output the second sub force moment

S240

In response to a transmission turntable moving to a second position, determine the second force moment that needs to be compensated when the drive turntable is at the second position

S250

Determine a fourth sub force moment that needs to be compensated by a second motor, based on the second force moment and a third sub force moment compensated by a second gravity compensation mechanism

S260

Drive the second motor to output the fourth sub force moment

FIG. 15

## INTERNATIONAL SEARCH REPORT

| International application No. |
|---|
| **PCT/CN2023/093226** |

**A. CLASSIFICATION OF SUBJECT MATTER**

A61B34/37(2016.01)i

According to International Patent Classification (IPC) or to both national classification and IPC

**B. FIELDS SEARCHED**

Minimum documentation searched (classification system followed by classification symbols)

IPC:A61B

Documentation searched other than minimum documentation to the extent that such documents are included in the fields searched

Electronic data base consulted during the international search (name of data base and, where practicable, search terms used)

CNTXT, CNABS, CNKI: 主手, 主操作, 主操控, 主操纵, 主控制, 重力, 平衡, 补偿; VEN, WOTXT, EPTXT, USTXT, GBTXT, CATXT, IEEE: main, master, manipulator, operation, handle, control, gravity, balance, compensate

**C. DOCUMENTS CONSIDERED TO BE RELEVANT**

| Category* | Citation of document, with indication, where appropriate, of the relevant passages | Relevant to claim No. |
|---|---|---|
| X | CN 110811843 A (SHANDONG UNIVERSITY) 21 February 2020 (2020-02-21) description, paragraphs 37-52, and figures 1-8 | 1-12, 20 |
| Y | CN 110811843 A (SHANDONG UNIVERSITY) 21 February 2020 (2020-02-21) description, paragraphs 37-52, and figures 1-8 | 13-15 |
| Y | CN 113715055 A (NINGBO RUIDA MEDICAL INSTRUMENT CO., LTD.) 30 November 2021 (2021-11-30) description, paragraphs 46-68, and figures 1-5 | 13-15, 16-19 |
| X | CN 108378922 A (HARBIN INSTITUTE OF TECHNOLOGY) 10 August 2018 (2018-08-10) description, paragraphs 33-68, and figures 1-16 | 1-12, 20 |
| Y | CN 108378922 A (HARBIN INSTITUTE OF TECHNOLOGY) 10 August 2018 (2018-08-10) description, paragraphs 33-68, and figures 1-16 | 13-19 |
| Y | CN 113017840 A (WUHAN UNITED IMAGING ZHIRONG MEDICAL TECHNOLOGY CO., LTD.) 25 June 2021 (2021-06-25) description, paragraphs 39-76, and figures 1-13 | 13-15, 16-19 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **28 July 2023** | **09 August 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/093226** |

**C. DOCUMENTS CONSIDERED TO BE RELEVANT**

| Category* | Citation of document, with indication, where appropriate, of the relevant passages | Relevant to claim No. |
| --- | --- | --- |
| Y | CN 113043279 A (NORCHANT INTELLIGENT MEDICAL TECHNOLOGY (HANGZHOU) CO., LTD.) 29 June 2021 (2021-06-29) description, paragraph 90, and figures 4-5 | 16-19 |
| A | CN 113662673 A (EDGE MEDICAL ROBOTICS CO., LTD.) 19 November 2021 (2021-11-19) entire document | 1-20 |
| A | CN 111067627 A (CHANGZHOU MAIKANGYI MEDICAL ROBOT CO., LTD.) 28 April 2020 (2020-04-28) entire document | 1-20 |
| A | US 2021145530 A1 (INTUITIVE SURGICAL OPERATIONS, INC.) 20 May 2021 (2021-05-20) entire document | 1-20 |

Form PCT/ISA/210 (second sheet) (July 2022)

**INTERNATIONAL SEARCH REPORT**
Information on patent family members

International application No.

**PCT/CN2023/093226**

| Patent document cited in search report | | | Publication date (day/month/year) | Patent family member(s) | | | Publication date (day/month/year) |
|---|---|---|---|---|---|---|---|
| CN | 110811843 | A | 21 February 2020 | CN | 110811843 | B | 25 January 2022 |
| CN | 113715055 | A | 30 November 2021 | CN | 216803503 | U | 24 June 2022 |
| CN | 108378922 | A | 10 August 2018 | None | | | |
| CN | 113017840 | A | 25 June 2021 | CN | 113017840 | B | 26 July 2022 |
| | | | | WO | 2022179638 | A1 | 01 September 2022 |
| CN | 113043279 | A | 29 June 2021 | CN | 113043279 | B | 02 August 2022 |
| CN | 113662673 | A | 19 November 2021 | None | | | |
| CN | 111067627 | A | 28 April 2020 | CN | 211355868 | U | 28 August 2020 |
| US | 2021145530 | A1 | 20 May 2021 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210656808X